# EUROPÄISCHE PATENTSCHRIFT

(11) **EP 2 239 249 B1**
(45) Veröffentlichungstag und Bekanntmachung des Hinweises auf die Patenterteilung: **10.05.2017**
(21) Anmeldenummer: 10003409.9
(22) Anmeldetag: 30.03.2010
(51) Int. Cl.: C07C 68/06, C07C 69/96

(54) **Verfahren zur Herstellung von Diaryl- oder Alkylarylcarbonaten aus Dialkylcarbonaten**
Method for producing diarylcarbonates or arylalkylcarbonates from dialkylcarbonates
Procédé de fabrication de carbonates de diaryle ou d'alkylaryle à partir de carbonates de dialkyle

(30) Priorität: 08.04.2009 DE 102009016853
(43) Veröffentlichungstag der Anmeldung: 13.10.2010
(73) Patentinhaber: Covestro Deutschland AG, 51373 Leverkusen (DE)
(72) Erfinder: Düx, Andre, 50321 Brühl (DE); Mothes, Helmut, Dr., 40764 Langenfeld (DE); Hallenberger, Kaspar, 51375 Leverkusen (DE); Ronge, Georg, Dr., 40549 Düsseldorf (DE); Warsitz, Rafael, 45136 Essen (DE); Ooms, Pieter, Dr., 47800 Krefeld (DE); Rechner, Johann, Dr., 47906 Kempen (DE)
(74) Vertreter: Levpat

(56) Entgegenhaltungen:
- EP-A2- 2 036 880
- JP-A- 2004 075 570
- IVO MUELLER AND EUGENY Y KENIG: "Reactive Distillation in a Dividing Wall Column: Rate-Based Modeling and Simulation" INDUSTRIAL & ENGINEERING CHEMISTRY RESEARCH, Bd. 46, Nr. 11, 28. April 2007 (2007-04-28), Seiten 3709-3719, XP007913880 ISSN: 0888-5885 [gefunden am 2007-04-28]

## Beschreibung

Gegenstand der Erfindung ist ein Verfahren zur Herstellung von Diarylcarbonaten und/oder Alkylarylcarbonaten aus Dimethylcarbonat und aromatischen Hydroxyverbindungen unter Einsatz einer reaktiven Trennwandkolonne.

Die Herstellung von aromatischen und aliphatisch-aromatischen Kohlensäurestern (Carbonaten) durch Umesterung ausgehend von aliphatischen Kohlensäurestern und aromatischen Hydroxyverbindungen ist im Prinzip bekannt. Dabei handelt es sich um eine Gleichgewichtsreaktion, wobei die Lage des Gleichgewichts fast vollständig in Richtung der aliphatisch substituierten Carbonate verschoben ist. Daher ist es verhältnismäßig leicht, aus aromatischen Carbonaten und Alkoholen aliphatische Carbonate herzustellen. Um die Reaktion jedoch im umgekehrten Sinne in Richtung aromatischer Carbonate durchzuführen, ist es notwendig, das sehr ungünstig liegende Gleichgewicht effektiv auf die Seite der aromatischen Carbonate zu verschieben, wobei nicht nur sehr aktive Katalysatoren, sondern auch geeignete Verfahrensführungen zur Anwendung gelangen müssen.

Es ist bekannt, derartige Gleichgewichtsreaktionen in Kolonnen durchzuführen und sie auf diese Weise vorteilhaft in Richtung der gewünschten Produktbildung zu verschieben, wie z.B. U. Block, Chem.-Ing. Techn. 49, 151 (1977), DE-OS 38 09 417, B. Schleper, B. Gutsche, J. Wnuck und L. Jeromin, Chem.-Ing.-Techn. 62, 226 (1990), Ullmans Encyclopädie der technischen Chemie, 4. Aufl., Bd. 3; S375 ff. 1973) beschreiben.

In den bekannten Verfahren erfolgt die Umesterung daher auch vorzugsweise kontinuierlich im Sinne einer Gegenstromumesterung in einer oder mehreren Reaktionskolonnen.

Die aus der Literatur bekannten Verfahren (z.B. EP 0 461 274, DE-A 42 26 755, DE-A 42 26 756) beschreiben jedoch meist nur diejenigen Verfahrensschritte, bei denen die Reaktion zum Diarylcarbonat durch Umesterung und/oder Disproportionierung stattfindet. Aktuelle Anmeldungen, wie beispielsweise WO 2006/033291 A1, EP 1 775 280A1, EP 1 767 516 A1, EP 1 767 517 A1, EP 1767 518 A1, EP 1 762 559 Alund EP 1 762 560 A1 geben zudem Hinweise bezüglich der apparativen Ausführungen von Reaktionskolonnen zur Herstellung von Diarylcarbonaten. Die in diesem Verfahren entstehenden Gemische aus Reaktionsalkohol und Dialkylcarbonat werden in von den Reaktionsapparaten getrennten Apparaten aufgetrennt.

Um die Wirtschaftlichkeit des Verfahrens gegenüber dem Stand der Technik zu erhöhen, sind sowohl Lösungsansätze zur Reduzierung des Energieverbrauchs als auch Maßnahmen zur Verringerung der Investitionskosten erforderlich. Erfahrungsgemäß können beide Anforderungen in der industriellen Praxis nur mit Einschränkungen erfüllt werden.

In EP-A 0 461 274 wird ein kontinuierlicher Umesterungsprozess zur Herstellung von aromatischen Carbonaten in einer oder in mehreren hintereinander geschalteten mehrstufigen Kolonnen beschrieben, wobei Dialkylcarbonate oder Alkylarylcarbonate mit Phenolen umgesetzt werden und am Kopf der Kolonnen die leichtflüchtigen Produkte, nämlich die Reaktionsalkohole und Dialkylcarbonate und am Sumpf der Kolonnen die schwersiedenden Produkte, wie z.B. Diarylcarbonate entnommen werden. Da sowohl Dialkylcarbonate als auch die Reaktionsalkhohole am Kopf der Kolonne entnommen werden ist zur Trennung dieser Komponenten mindestens ein weiterer Schritt erforderlich.

DE-A 42 26 756 beschreibt ein zweistufiges Verfahren zur Herstellung von Diarylcarbonaten durch Umesterung eines Dialkylcarbonates mit einer aromatischen Hydroxyverbindung, bei dem in einer ersten Stufe aus den Ausgangsstoffen zunächst das korrespondierende Alkylarylcarbonat und in einer zweiten Stufe das Diarylcarbonat gebildet wird. Die Angaben in der Verfahrensbeschreibung beschränken sich dabei auf die Reaktionsbedingungen, den verwendeten Katalysator und die Ausführung der Reaktionskolonnen. Auch im Falle dieser Anmeldung werden Reaktionsalkohol und Diakylcarbonat am Kopf der Kolonnen entnommen.

DE-A 42 26 755 beschreibt ein Verfahren zur Herstellung von Diarylcarbonaten in zwei energetisch und stofflich miteinander gekoppelten Reaktionskolonnen, wobei in der ersten Stufe eine aromatische Hydroxyverbindungen und ein Dialkylcarbonat, zur Reaktion gebracht werden und das dabei gebildete Alkylarylcarbonat in der zweiten Stufe entweder durch Umesterung mit der aromatischen Hydroxyverbindung oder durch Disproportionierung zum Diarylcarbonat umgesetzt wird. Problematisch ist hierbei allerdings, dass durch die stoffliche und energetische Integration des Verfahrens die Reaktionsbedingungen für die Bildung des Alkylaryl- bzw. Diarylcarbonats nicht optimal gewählt werden können, da diese durch den in beiden Schritten vorliegenden und nahezu identischen Druck festgelegt sind.

Die EP-A 781 760 beschreibt ein kontinuierliches Verfahren zur Herstellung aromatischer Carbonate durch Reaktion eines Dialkylcarbonates mit einer aromatischen Hydroxyverbindung in Anwesenheit eines Katalysators, kontinuierlicher Entfernung des bei der Reaktion entstehenden aromatischen Carbonats, der alkoholischen Nebenprodukte, des Dialkylcarbonats und der aromatischen Hydroxyverbindung, wobei das Dialkylcarbonat und die aromatische Hydroxyverbindung wieder in die Reaktion zurückgeführt werden. Auch bei diesem Verfahren erhält man im Verfahrensabschnitt Reaktion lediglich zwei Fraktionen, die am Oberen Ende bzw. am Boden des Reaktors entnommen werden.

WO-A 2006/001256 beschreibt ein Verfahren, bei dem eine aromatische Hydroxyverbindung mit einem Dialkylcarbonat in Anwesenheit eines Katalysators umgesetzt wird sowie eine hierzu geeignete technische Vorrichtung. Auch hier erfolgt die Entnahme der Fraktionen nur als Kopf oder Sumpfprodukt.

Aus der EP 2 036 880 A2 ist ein Verfahren zur Herstellung von Diaryl- oder Alkylarylcarbonaten aus Dialkylcarbonaten bekannt, wobei wenigstens zwei Reaktionskolonnen verwendet werden, wobei die Kondensationswärme einer oder mehrerer dieser Kolonnen in anderen Verfahrensschritten dieses Verfahrens genutzt wird. Ohne entsprechend effiziente apparative und energetische Integration sind die Investitions- und Energiekosten der vorangehend beschriebenen Verfahren bekanntermaßen hoch, was wiederum die Vorteilhaftigkeit der phosgenfreien Herstellung von Arylcarbonaten aus ökologischer und ökonomischer Hinsicht in Frage stellt.

Ansätze für eine apparative und energetische Integration ergeben sich durch die Verwendung von Trennwandkolonnen.

Ein Beispiel für die Funktionsweise von Trennwandkolonne bei reaktiven Trennverfahren wird in der EP 0 126 288B1 beschrieben. Allerdings werden hier keine Hinweise gegeben, wie sich diese Technologie bei der Herstellung von Diarylcarbonaten durch Umesterung eines Dialkylcarbonates anwenden lässt.

Die JP 2004 075570 A offenbart die Synthese von Diphenylcarbonat ausgehend von Dimethylcarbonat und Phenol, wobei der Reaktion durch mehrere in Längsrichtung wirkende Trennwände geteilt ist.

Ein weiteres Beispiel für einen reaktiven Trennprozess findet man in Ind. Eng. Chem. Res. 2007, 46, 3709-3719. In dieser Offenlegung wird eine Umesterung von Dimethylcarbonat mit Ethanol zum aliphatischen Diethylcarbonat beschrieben. Bei diesem Verfahren wird also ebenfalls kein Diarylcarbonat erzeugt.

Bei der Umesterung einer aromatischen Hydroxyverbindung mit einem Alkylcarbonat wird in der Regel letzteres zur Erzielung eines hohen Umsatzes bezogen auf die aromatische Hydroxyverbindung im Überschuss gefahren.

Im Falle einer Umesterung in einer Reaktionskolonne wird dabei am Kopf der Kolonne sowohl das im Überschuss eingesetzte Dialkylcarbonat als auch der bei der Reaktion anfallende Reaktionsalkohol abgetrennt. Im Sumpfprodukt ist bevorzugt die aromatische Hydroxyverbindung das bei der Reaktion gebildete Alkylarylcarbonat gegebenenfalls Diarylcarbonat und Dialkylcarbonat enthalten. Im Falle einer homogen katalysierten Reaktion enthält das Sumpfprodukt auch den Katalysator.

Nachteilhaft bei dieser Fahrweise ist, dass das im Überschuss eingesetzte Dialkylcarbonat in einem separaten Schritt vom Reaktionsalkohol abgetrennt werden muss.

Eine Trennung von Reaktionsalkohol vom Dialkylcarbonat in einem Verstärkungsteil oberhalb der Reaktionszone ist zwar prinzipiell möglich, aber im Hinblick auf den Umsatz wenig effektiv, da es somit nur von oben auf die Reaktionszone aufgegeben wird. Auf diese Weise wird lediglich die Trennung von Dialkylcarbonat und aromatischer Hydroxyverbindung verstärkt. Dieser Effekt sollte jedoch vermieden werden um einem hohen Umsatz an aromatischer Hydroxyverbindnung sicherstellen zu können.

Es bestand folglich weiterhin Bedarf, ein Verfahren zur Herstellung von aromatischen Carbonaten, d.h. Diaryl- und/oder Alkylarylcarbonaten, vorzugsweise Diarylcarbonaten, bereitzustellen, welches die vorangehend genannten Nachteile nicht aufweist und in welchem gegenüber den vorangehend genannten bekannten Verfahren eine energetisch und apparativ integrierten Prozess bereitzustellen.

Die Aufgabe, die der Erfindung zugrunde lag, bestand demnach darin, ein Verfahren zur Herstellung von aromatischen Carbonaten, d.h. Diaryl- und/oder Alkylarylcarbonaten, bereitzustellen, in welchem gegenüber bekannten Verfahren die Reaktion des Dialkylcarbonats mit der aromatischen Hydroxyverbindung und die destillative Trennung des Gemisches aus Reaktionsalkohol und Dialkylcarbonat apparativ und energetisch gekoppelt sind.

Es wurden nun gefunden dass die Abtrennung des bei der Reaktion anfallenden Reaktionsalkohols und die des im Überschuss eingesetzten Dialkylcarbonats in einer Reaktionskolonne erfolgen kann, ohne die o.g. Nachteile in Kauf nehmen zu müssen.

Dies gelingt überraschenderweise unter Verwendung einer reaktiven Trennwandkolonne (RTWK).

Die Erfindung betrifft demzufolge Verfahren zur Herstellung von Alkylarylcarbonaten und/oder Diarylcarbonaten aus Dimethylcarbonat und aromatischen Hydroxyverbindungen in Gegenwart eines Katalysators mittels einer Reaktivdestillationskolonne, die oberhalb des Abtriebsteils (K₁AT) und unterhalb eines Verstärkungsteils (K₁VT) durch in Längsrichtung verlaufende Trennwand (T), die eine Quervermischung von Flüssigkeits- und/oder Brüdenströmen ganz oder teilweise verhindern, in eine Zulaufseite (Z), in der sich eine Reaktionszone befindet, und eine Entnahmeseite (E) unterteilt ist, dadurch gekennzeichnet, dass der Zulaufseite ein die aromatische Hydroxyverbindung enthaltender Strom (21) und ein das Dimethylcarbonat enthaltender Strom (22) zugeführt werden und aus der Entnahmeseite ein Dimethylcarbonat-haltiger Seitenstrom (5) flüssig entnommen, ggf. mit einem weiteren Dimethylcarbonat-haltigen Strom (2) vermischt, und anschließend der Reaktionszone auf der Zulaufseite der reaktiven Trennwandkolonne zugeführt wird und am Kopf das Dampfgemisch enthaltend Dimethylcarbonat und während der Reaktion gebildetem Alkylalkohol und in einer Membrantrennung aufgetrennt wird.

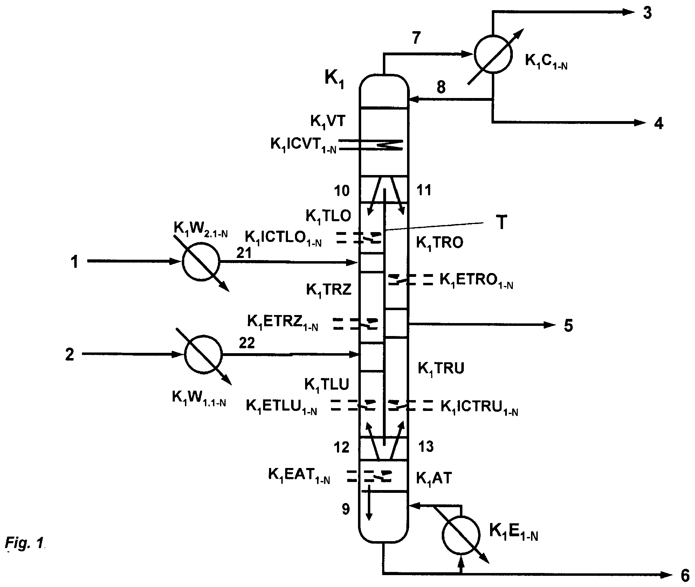

Der Aufbau einer solchen reaktiven Trennwandkolonne wird in Fig. 1 dargestellt.

Die Kolonne (K₁) wird oberhalb des Abtriebsteils (K₁AT) und unterhalb eines Verstärkungsteils (K₁VT) durch eine in Längsrichtung verlaufende Trennwand (T) in eine Zulaufseite und eine Entnahmeseite aufgeteilt.

Auf der Zulaufseite befindet sich eine Reaktionszone (K₁TRZ). In einer bevorzugten Aufführungsform wird ein die aromatische Hydroxyverbindung und gegebenenfalls den Umesterungskatalysator enthaltender Strom (21) oberhalb dieser Reaktionszone und ein, das Dimethylcarbonat enthaltender Strom (22) unterhalb der Reaktionszone bevorzugt dampfförmig oder erhitzt zugeführt.

Der die aromatische Hydroxyverbindung und gegebenenfalls den Katalysator enthaltende Strom wird vorzugsweise flüssig oder nur mit geringem Dampfanteil eindosiert, wobei der Gasanteil bevorzugt weniger als 20 Gew.-% beträgt.

Der Dimethylcarbonat-haltige Strom wird bevorzugt dampfförmig oder überhitzt zugeführt. In bevorzugten Ausführungsformen kann die Überhitzung des Dampfstroms 0 bis 50 °C betragen.

Im Rahmen der Erfindung hergestellte Diarylcarbonate sind bevorzugt solche der allgemeinen Formel (I) 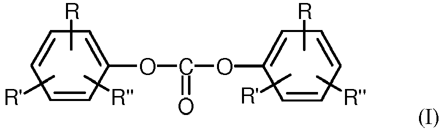 wobei R, R' und R" unabhängig voneinander H, lineares oder verzweigtes, gegebenenfalls substituiertes C₁-C₃₄-Alkyl, bevorzugt C₁-C₆-Alkyl, besonders bevorzugt C₁-C₄-Alkyl, C₁-C₃₄-Alkoxy, bevorzugt C₁-C₆-Alkoxy, besonders bevorzugt C₁-C₄-Alkoxy, C₅-C₃₄-Cycloalkyl, C₇-C₃₄-Alkylaryl, C₆-C₃₄-Aryl oder einen Halogenrest, bevorzugt einen Chlorrest darstellen und R, R' und R" auf beiden Seiten der Formel (I) gleich oder verschieden sein können. R kann auch -COO-R"' bedeuten, wobei R'" H, gegebenenfalls verzweigtes C₁-C₃₄ Alkyl, bevorzugt C₁-C₆-Alkyl, besonders bevorzugt C₁-C₄-Alkyl, C₁-C₃₄-Alkoxy, bevorzugt C₁-C₆-Alkoxy, besonders bevorzugt C₁-C₄-Alkoxy, C₅-C₃₄-Cycloalkyl, C₇-C₃₄-Alkylaryl oder C₆-C₃₄-Aryl sein kann. Bevorzugt sind R, R' und R" auf beiden Seiten der Formel (I) gleich. Ganz besonders bevorzugt stehen R, R' und R" für H.

Diarylcarbonate der allgemeinen Formel (I) sind beispielsweise: Diphenylcarbonat, Methylphenylphenyl-carbonate und Di-(methylphenyl)-carbonate, auch als Gemisch, wobei die Stellung der Methylgruppe an den Phenylringen beliebig sein kann, sowie Dimethylphenyl-phenyl-carbonate und Di-(dimethylphenyl)-carbonate, auch als Gemisch, wobei die Stellung der Methylgruppen an den Phenylringen beliebig sein kann, Chlorphenyl-phenyl-carbonate und Di-(chlorphenyl)-carbonate, wobei die Stellung der Methylgruppe an den Phenylringen beliebig sein kann, 4-Ethylphenyl-phenyl-carbonat, Di-(4-ethylphenyl)-carbonat, 4-n-Propylphenyl-phenyl-carbonat, Di-(4-n-propylphenyl)-carbonat, 4-iso-Propylphenyl-phenyl-carbonat, Di-(4-iso-propylphenyl)-carbonat, 4-n-Butylphenyl-phenyl-carbonat, Di-(4-n-butylphenyl)-carbonat, 4-iso-Butylphenyl-phenyl-carbonat, Di-(4-iso-butylphenyl)-carbonat, 4-tert-Butylphenyl-phenyl-carbonat, Di-(4-tert-butylphenyl)-carbonat, 4-n-Pentylphenyl-phenyl-carbonat, Di-(4-n-pentylphenyl)-carbonat, 4-n-Hexylphenyl-phenyl-carbonat, Di-(4-n-hexylphenyl)-carbonat, 4-iso-Octylphenyl-phenyl-carbonat, Di-(4-iso-octylphenyl)-carbonat,4-n-Nonylphenyl-phenyl-carbonat, Di-(4-n-nonylphenyl)-carbonat, 4-Cyclohexylphenyl-phenyl-carbonat, Di-(4-cyclohexylphenyl)-carbonat, 4-(1-Methyl-1-phenylethyl)-phenyl-phenyl-carbonat, Di-[4-(1-methyl-1-phenylethyl)-phenyl]-carbonat, Biphenyl-4-yl-phenyl-carbonat, Di-(biphenyl-4-yl)-carbonat, (1-Naphthyl)-phenyl-carbonat, (2-Naphthyl)-phenyl-carbonat, Di-(1-naphthyl)-carbonat, Di-(2-naphthyl)-carbonat, 4-(1-Naphthyl)-phenyl-phenyl-carbonat, 4-(2-Naphthyl)-phenyl-phenyl-carbonat, Di-[4-(1-naphthyl)phenyl]-carbonat, Di-[4-(2-naphthyl)phenyl]-carbonat, 4-Phenoxyphenyl-phenyl-carbonat, Di-(4-phenoxyphenyl)-carbonat, 3-Pentadecylphenyl-phenyl-carbonat, Di-(3-pentadecylphenyl)-carbonat, 4-Tritylphenylphenyl-carbonat, Di-(4-tritylphenyl)-carbonat, Methylsalicylat-phenyl-carbonat, Di-(methylsalicylat)-carbonat, Ethylsalicylat-phenyl-carbonat, Di-(ethylsalicylat)-carbonat, n-Propylsalicylat-phenyl-carbonat, Di-(n-propylsalicylat)-carbonat, iso-Propylsalicylat-phenyl-carbonat, Di-(iso-propylsalicylat)-carbonat, n-Butylsalicylat-phenyl-carbonat, Di-(n-butylsalicylat)-carbonat, iso-Butylsalicylat-phenyl-carbonat, Di-(iso-butylsalicylat)-carbonat, tert-Butylsalicylat-phenyl-carbonat, Di-(tert-butylsalicylat)-carbonat, Di-(phenylsalicylat)-carbonat und Di-(benzylsalicylat)-carbonat.

Bevorzugte Diarylcarbonate sind: Diphenylcarbonat, 4-tert-Butylphenyl-phenyl-carbonat, Di-(4-tert-butylphenyl)-carbonat, Biphenyl-4-yl-phenyl-carbonat, Di-(biphenyl-4-yl)-carbonat, 4-(1-Methyl-1-phenylethyl)-phenyl-phenyl-carbonat und Di-[4-(1-methyl-1-phenylethyl)-phenyl]-carbonat.

Besonders bevorzugt ist Diphenylcarbonat.

Im Rahmen der Erfindung eingesetzte Dialkylcarbonate sind Dimethylcarbonat.

Im Rahmen der Erfindung geeignete aromatische Hydroxyverbindungen sind bevorzugt solche der allgemeinen Formel (III) 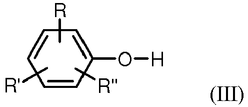 worin R, R' und R" unabhängig voneinander die für die allgemeine Formel (I) genannte Bedeutung haben können.

Solche aromatischen Hydroxyverbindungen sind beispielsweise: Phenol, o-, m- oder p-Kresol, auch als Gemisch der Kresole, Dimethylphenol, auch als Gemisch, wobei die Stellung der Methylgruppen am Phenolring beliebig sein kann, z.B. 2,4-, 2,6-, oder 3,4-Dimethylphenol, o-, m- oder p-Chlorphenol, o-, m- oder p-Ethylphenol, o-, m- oder p-n-Propylphenol, 4-iso-Propylphenol, 4-n-Butylphenol, 4-iso-Butylphenol, 4-tert-Butylphenol, 4-n-Pentylphenol, 4-n-Hexylphenol, 4-iso-Octylphenol, 4-n-Nonylphenol, o-, m- oder p-Methoxyphenol, 4-Cyclohexylphenol, 4-(1-Methyl-1-phenylethyl)-phenol, Biphenyl-4-ol, 1-Naphthol, 2-1-Naphthol, 4-(1-Naphthyl)phenol, 4-(2-Naphthyl)-phenol, 4-Phenoxyphenol, 3-Pentadecylphenol, 4-Tritylphenol, Methylsalicylsäure, Ethylsalicylsäure, n-Propylsalicylsäuret, iso-Propylsalicylsäure, n-Butylsalicylsäure, iso-Butylsalicylsäure, tert-Butylsalicylsäure, Phenylsalicylsäure und Benzylsalicylsäure.

Bevorzugte aromatischen Hydroxyverbindungen sind Phenol, 4-tert-Butylphenol, Biphenyl-4-ol und 4-(1-Methyl-1-phenylethyl)-phenol.

Besonders bevorzugt ist Phenol.

Im Rahmen der Erfindung hergestellte Alkylarylcarbonate sind bevorzugt solche der allgemeinen Formel (IV) 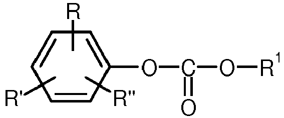 worin R, R' und R" die für die allgemeine Formel (I) genannte Bedeutung haben können und R¹ Methyl ist.

Bevorzugte Alkylarylcarbonate sind Methyl-phenyl-carbonat, Methyl-(o-kresyl)-carbonat, Methyl-(p-kresyl)-carbonat, Methyl(p-chlorphenyl)-carbonat. Ein besonders bevorzugtes Alkylarylcarbonat ist Methyl-phenyl-carbonat

Sowohl die für das erfindungsgemäße Verfahren geeigneten Dialkylcarbonate als auch die aromatischen Hydroxyverbindungen sind dem Fachmann bekannt und kommerziell erhältlich oder können nach dem Fachmann ebenfalls bekannten Verfahren hergestellt werden.

**C₁-C₄-Alkyl** steht im Rahmen der Erfindung beispielsweise für Methyl, Ethyl, n-Propyl, iso-Propyl, n-Butyl, sec.-Butyl, tert.-Butyl, **C₁-C₆-Alkyl** darüber hinaus beispielsweise für n-Pentyl, 1-Methylbutyl, 2-Methylbutyl, 3-Methylbutyl, neo-Pentyl, 1-Ethylpropyl, Cyclohexyl, Cyclopentyl, n-Hexyl, 1,1-Dimethylpropyl, 1,2-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl oder 1-Ethyl-2-methylpropyl, **C₁-C₃₄-Alkyl** darüber hinaus beispielsweise für n-Heptyl und n-Octyl, Pinakyl, Adamantyl, die isomeren Menthyle, n-Nonyl, n-Decyl, n-Dodecyl, n-Tridecyl, n-Tetradecyl, n-Hexadecyl oder n-Octadecyl. Gleiches gilt für den entsprechenden Alkylrest beispielsweise in Aralkyl- bzw. Alkylarylresten. Alkylenreste in den entsprechenden Hydroxyalkyl- oder Aralkyl- bzw. Alkylarylresten stehen beispielsweise für die den vorangehenden Alkylresten entsprechenden Alkylenreste.

**Aryl** steht für einen carbocyclischen aromatischen Rest mit 6 bis 34 Gerüstkohlenstoffatomen. Gleiches gilt für den aromatischen Teil eines Arylalkylrestes, auch Aralkylrest genannt, sowie für Arylbestandteile komplexerer Gruppen, wie z.B. Arylcarbonylresten.

**Arylalkyl** bzw. **Aralkyl** bedeutet jeweils unabhängig einen geradkettigen, cyclischen, verzweigten oder unverzweigten Alkyl-Rest nach vorstehender Definition, der einfach, mehrfach oder vollständig durch Aryl-Reste gemäß vorstehender Definition substituiert sein kann.

Die vorangehenden Aufzählungen sind beispielhaft und nicht als Limitierung zu verstehen.

Im erfindungsgemäßen Verfahren werden die aromatische(n) Hydroxyverbindung(en) und das oder die Dimethylcarbonat(e) bevorzugt im Molverhältnis von 1 : 0.1 bis 1 : 10, besonders bevorzugt von 1 : 0.2 bis 1 : 5, ganz besonders bevorzugt von 1 : 0.5 bis 1 : 3 eingesetzt. Dabei berücksichtigt das angegebene Molverhältnis nicht die Rückführung von aromatischer Hydroxyverbindung oder Dimethylcarbonat in die Umesterungskolonne über einen oder mehrere Kopfkondensator(en) (vgl. unter (b)) oder einen oder mehrere etwaig vorhandene(n) Sumpfverdampfer. (Verhältnis bezieht sich auf die in Summe in den Strömen 21 und 22 enthaltenen Mengen an Dimethylcarbonat und aromatischer Hydroxyverbindung)

Der das Dimethylcarbonat enthaltende Strom (22) kann, insbesondere bei kontinuierlich geführten Verfahren, neben dem Dimethylcarbonat auch Teile der aromatischen Hydroxyverbindung, die bei der Reaktion anfallende aliphatische Hydroxyverbindung R¹-OH und/oder R²-OH (Reaktionsalkohol), sehr geringe Mengen des bei der Umesterung anfallenden Alkylarylcarbonats und/oder Diarylcarbonats und bei der Reaktion entstehende unerwünschte Nebenkomponenten enthalten. Der das Dimethylcarbonat enthaltende Strom (22) kann beispielsweise 0 bis 5 Gew.-%, bevorzugt 0.05 bis 3 Gew: % und besonders bevorzugt 0.05 bis 2 Gew: % des Reaktionsalkohols, 0 bis 40 Gew.-%, bevorzugt 0 bis 10 Gew.-%, besonders bevorzugt 0 bis 5 Gew.-% der aromatischen Hydroxyverbindung, 0 bis 5 Gew.-% Alkylarylcarbonat, 0 bis 5 Gew.-% Diarylcarbonat und 0 bis 5 Gew.-% sonstige bei der Reaktion anfallende Nebenverbindungen (wie z.B. Alkylarylether) oder bereits in den Edukten enthaltene Verunreinigungen, jeweils bezogen auf das Gesamtgewicht des Dimethylcarbonat enthaltenden Stromes, aufweisen. Vorzugsweise enthält der das Dimethylcarbonat enthaltende Strom (22) 50 bis 100 Gew.-% Dimethylcarbonat, bezogen auf das Gesamtgewicht des Dimethylcarbonat enthaltenden Stromes, wobei sich die Anteile der einzelnen vorangehend genannten Komponenten zu 100 Gew.-% addieren. Der die aromatische Hydroxyverbindung enthaltende Strom (21) kann, insbesondere bei kontinuierlich geführten Verfahren, neben der aromatischen Hydroxyverbindung auch Teile des Dimethylcarbonats, das bei der Umesterung anfallende Alkylarylcarbonat und/oder Diarylcarbonat, in sehr geringen Mengen den Reaktionsalkohol und bei der Reaktion entstehende unerwünschte Nebenprodukte enthalten. Beispielsweise kann der Gehalt des Dimethylcarbonats 0 bis 50 Gew.-%, der Gehalt des Reaktionsalkohols 0 bis 10 Gew.-%, bevorzugt 0 bis 5 Gew.-%, der Gehalt des Alkylarylcarbonats und des Diarylcarbonats jeweils 0 bis 10 Gew.-%, bevorzugt 0 bis 5 Gew.-% und der Gehalt der unerwünschten Nebenprodukte 0 bis 5 Gew.-%, bevorzugt 0 bis 1 Gew.-%, jeweils bezogen auf das Gesamtgewicht des die aromatische Hydroxyverbindung enthaltenden Stromes, betragen. Mit dem die aromatische Hydroxyverbindung enthaltenden Strom (21) kann zudem der Katalysator in die Umesterungskolonne eingespeist werden. In diesem Fall beträgt der Gehalt an Katalysator bevorzugt 0 bis 5 Gew.-%, bezogen auf das Gesamtgewicht des die aromatische Hydroxyverbindung enthaltenden Stromes. Vorzugsweise enthält der die aromatische Hydroxyverbindung enthaltende Strom (21) 50 bis 100 Gew.-% aromatische Hydroxyverbindung, bezogen auf das Gesamtgewicht des die aromatische Hydroxyverbindung enthaltenden Stromes, wobei sich die Anteile der einzelnen vorangehend genannten Komponenten zu 100 Gew.-% addieren.

Für die in der Trennwandkolonne auftretenden Reaktionsschritte können aus der Literatur bekannte Umesterungskatalysatoren eingesetzt werden. Dies sind aus der Literatur bekannte Umesterungskatalysatoren für die Dialkylcarbonat-Phenol-Umesterung, wie AlX₃, TiX₃, UX₄, TiX₄, VOX₃, VX₅, ZnX₂, FeX₃, PbX₂ und SnX₄, worin X für Halogen-, Acetoxy-, Alkoxy- oder Aryloxyreste steht (DE-OS 2 58 412). Besonders bevorzugte erfindungsgemäß einsetzbare Katalysatoren sind Metallverbindungen wie AlX₃, TiX₄, PbX₂ und SnX₄, wie beispielsweise Titantetrachlorid, Titantetramethoxid Titantetraphenoxid, Titantetraethoxid, Titantetraisopropylat, Titantetradodecylat, Zinntetraisooctylat und Aluminiumtriisopropylat. Ganz besonders bevorzugt sind Metallverbindungen TiX₄. Die genannten Metallverbindungen werden bevorzugt in Mengen von 0,001 bis 5 Gew.-%, bevorzugt von 0,005 bis 5 Gew.-% und besonders bevorzugt von 0,01 bis 5 Gew.-%, bezogen auf das Gewicht des umzusetzenden Reaktionsgemischs, eingesetzt.

Halogen bedeutet im Rahmen der Erfindung Fluor, Chlor oder Brom, bevorzugt Fluor oder Chlor, besonders bevorzugt Chlor.

Weitere erfindungsgemäß einsetzbare Katalysatoren sind zinnorganische Verbindungen der allgemeinen Formel (R¹¹)₄₋ₓ-Sn(Y)ₓ, in der Y für einen Rest OCOR¹², OH oder OR steht, wobei R¹² C₁-C₁₂-Alkyl, C₆-C₁₂-Aryl oder C₇-C₁₃-Alkylaryl bedeutet, R¹¹ unabhängig von R¹² die Bedeutung von R¹² hat und x eine ganze Zahl von 1 bis 3 bedeutet, Dialkylzinnverbindungen mit 1 bis 12 C-Atomen im Alkylrest oder Bis-(trialkylzinn)verbindungen, beispielsweise Trimethylzinnacetat, Triethylzinnbenzoat, Tributylzinnacetat, Triphenylzinnacetat, Dibutylzinndiacetat, Dibutylzinndilaurat, Dioctylzinndilaurat, Dibutylzinnadipinat, Dibutyldimethoxyzinn, Dimethylzinnglykolat, Dibutyldiethoxyzinn, Triethylzinnhydroxid, Hexaethylstannoxan, Hexabutylstannoxan, Dibutylzinnoxid, Dioctylzinnoxid, Butylzinntriisooctylat, Octylzinntriisooctylat, Butylstannonsäure und Octylstannonsäure in Mengen von 0,001 bis 20 Gew: % (vgl. EP 879, EP 880, EP 39 452, DE-OS 34 45 555, JP 79/63023), polymere Zinnverbindungen der Formel -[-RR¹¹Sn-O-]-, in welcher R und R¹¹ unabhängig voneinander die vorangehend für R¹² genannte Bedeutung haben, beispielsweise Poly[oxy(dibutylstannylen)] Poly[oxy(dioctylstannylen)], Poly[oxy(butylphenylstannylen)] und Poly[oxy(diphenylstannylen)] (DE-OS 34 45 552), polymere Hydroxystannoxane der Formel -[-RSn(OH)-O-]-, beispielsweise Poly(ethylhydroxystannoxan), Poly(butylhydroxystannoxan), Poly-(octylhydroxystannoxan), Poly(undecylhydroxystannoxan) und Poly(dodecylhydroxystannoxane) in Mengen von 0,001 bis 20 Gew.-%, bevorzugt von 0,005 bis 5 Gew.-%, bezogen auf Dialkylcarbonat (DE-OS 40 06 520). Weitere erfindungsgemäß einsetzbare Zinnverbindungen sind Sn(II)oxide der allgemeinen Formel

X-R₂Sn-O-R₂Sn-Y,

worin X und Y unabhängig voneinander OH, SCN, OR¹³, OCOR¹³ oder Halogen und R Alkyl, Aryl bedeuten soll, worin R¹³ die vorangehend für R¹² genannte Bedeutung hat (EP 0 338 760).

Als weitere erfindungsgemäß einsetzbare Katalysatoren kommen Bleiverbindungen, gegebenenfalls zusammen mit Triorganophosphanen, einer Chelatverbindung oder einem Alkalimetallhalogenid, beispielsweise Pb(OH)₂-2PbCO₃, Pb(OCO-CH₃)₂, Pb(OCO-CH₃)₂ ·2LiCl, Pb(OCO-CH₃)₂ • 2PPh3 in Mengen von 0,001 bis 1, bevorzugt von 0,005 bis 0,25 Mol pro Mol Dialkylcarbonat (JP 57/176932, JP 01/093580), sowie andere Blei(II)- und Blei(IV)-verbindungen, wie PbO, PbO₂, Mennige, Plumbite, und Plumbate (JP 01/093560), Eisen(III)acetat (JP 61/1 72 852), weiterhin Kupfersalze und/oder Metallkomplexe, beispielsweise von Alkali, Zink, Titan und Eisen (JP 89/005588).

Weiterhin sind in den erfindungsgemäßen Verfahren heterogene Katalysatorsysteme einsetzbar. Solche sind beispielsweise Mischoxide aus Silizium und Titan, die durch gemeinsame Hydrolyse von Silizium und Titanhalogeniden erhältlich sind (JP 54/125617) oder Titandioxide mit hoher BET-Oberfläche >20 m²/g (DE-OS 40 36 594)).

Bevorzugte Katalysatoren für das erfindungsgemäße Verfahren sind die vorangehend genannten Metallverbindungen AlX₃, TiX₃, UX₄, TiX₄, VOX₃, VX₅, ZnX₂, FeX₃, PbX₂ und SnX₄. Besonders bevorzugt sind AlX₃, TiX₄, PbX₂ und SnX₄, wovon beispielhaft Titantetrachlorid, Titantetramethoxid Titantetraphenoxid, Titantetraethoxid, Titantetraisopropylat, Titantetradodecylat, Zinntetraisooctylat und Aluminiumtriisopropylat genannt seien. Ganz besonders bevorzugt sind Metallverbindungen TiX₄. Insbesondere bevorzugt sind Titantetramethoxid, Titantetraphenoxid und Titantetraethoxid.

Der Katalysator wird bevorzugt zusammen mit dem Strom enthaltend die aromatische(n) Hydroxyverbindung(en) in gelöster oder suspendierter Form in die erste Reaktionskolonne eingebracht. Alternativ kann der Katalysator auch separat beispielsweise in einem dem Reaktionsalkohol entsprechenden Alkohol oder einem geeigneten inerten Lösungsmittel zudosiert werden. Im Falle der Verwendung heterogener Katalysatoren können diese im Gemisch mit den genannten Füllkörpern, in geeigneter Form anstelle von Füllkörpern oder als Schüttung auf etwaig eingebaute Kolonnenböden eingesetzt werden.

Der in der Reaktionszone vorgesehene Flüssigkeitshold-up wird so bemessen, dass die Verweilzeit der durch diese Reaktionszone laufende Flüssigkeit zwischen 1 und 120 min, bevorzugt 10 bis 60 und besonders bevorzugt 15 bis 40 min beträgt.

Zur Einstellung dieser Verweilzeit können Packungen, Füllkörper oder Böden oder Kombinationen aus verschiedenen Einbauten (z.B. Packungen und Verweilzeitböden) eingesetzt werden. Bevorzugt werden Böden eingesetzt. Besonders bevorzugt werden Böden mit einem hohen Flüssigkeitsgehalt verwendet. Letzterer wird durch den Stand der Flüssigkeit beeinflusst. Der Stand der unbegasten Flüssigkeit auf den Böden liegt zwischen 20 und 400 mm, bevorzugt zwischen 40 und 300 und besonders bevorzugt zwischen 80 und 250 mm.

Die Anzahl der theoretischen Trennstufen in der Reaktionszone liegt unabhängig von der Art der gewählten Einbauten zwischen 5 und 100, bevorzugt zwischen 10 und 60 und besonders bevorzugt zwischen 20 und 40.

Die Reaktionszone kann im Falle einer heterogen katalysierten Reaktion den Katalysator enthalten. Darüber hinaus können Zwischenverdampfer (K₁ETRZ_{1-N}) zur gezielten Einstellung der gewünschten Reaktionstemperatur integriert werden.

Die Reaktion erfolgt in einem Bereich von 100 bis 300 °C, bevorzugt 150 - 280 °C und besonders bevorzugt von 180 bis 245 °C. Der Druck der Reaktionszone liegt bevorzugt im Bereich von 0.5 bis 20 bar, besonders bevorzugt von 1 bis 15 bar, ganz besonders bevorzugt von 2 bis 10 bar.

Das am Kopf der Trennwandkolonne entnommene Dampfgemisch enthaltend Dimethylcarbonat und während der Reaktion gebildetem Alkylalkohol bevorzugt nach Kondensation am Kopf der Trennwandkolonne ganz oder teilweise wenigstens einem weiteren Verfahrensschritt der Membrantrennungzur Trennung von Dialkylcarbonat und Alkylalkohol zugeführt.

Die Trennung des Dimethylcarbonats und des Reaktionsalkohols erfolgt bevorzugt in einer Kombination aus Destillation und Membrantrennung - im Folgenden als Hybridprozess - bezeichnet.

Bilden Reaktionsalkohol und Dialkylcarbonat ein Azeotrop (z.B. Methanol und Dimethylcarbonat) so wird bevorzugt ein wenigstens zweistufiges Verfahren wie beispielsweise ein Zweidruckverfahren, eine Extraktivdestillation, eine Heteroazeotropdestillation mit einem niedrigsiedenden Schleppmittel oder ein Hybridverfahren verwendet. Besonders bevorzugt wird das Zweidruckverfahren oder ein Hybridverfahren angewendet. Ganz besonders bevorzugt wird das Zweidruckverfahren angewendet. Solche Verfahren sind dem Fachmann grundsätzlich bekannt (vgl. z.B. Ullmann's Encyclopedia of Industrial Chemistry, Vol. 7, 2007, Kap. 6.4. und 6.5.; Chemie Ingenieur Technik (67) 11 / 95).

Bilden Reaktionsalkohol und Dialkylcarbonat kein Azeotrop (z.B. Ethanol und Diethylcarbonat), so erfolgt die Trennung bevorzugt in einer einzelnen Destillationskolonne.

Bilden Reaktionsalkohol und Dialkylcarbonat ein Azeotrop, so weist das Destillat einer ersten Destillationskolonne des Verfahrensschritts zur Trennung von Dialkylcarbonat und Alkylalkohol (Reaktionsalkohol) vorzugsweise nahezu azeotrope Zusammensetzung auf. In diesem Fall wird dieses bevorzugt in einem Zweidruckverfahren wenigstens einer weiteren Destillationskolonne zugeführt die bei einem Betriebsdruck arbeitet, der unter dem der ersten Destillationskolonne liegt. Durch den unterschiedlichen Betriebsdruck verschiebt sich die Lage des Azeotrops hin zu geringeren Anteilen an Reaktionsalkohol. Man erhält als Sumpfprodukt dieser zweiten oder weiteren Destillationskolonne(n) Reaktionsalkohol in einer Reinheit von 90 bis 100 Gew.-%, bezogen auf das Gesamtgewicht des isolierten Sumpfproduktes, und als Destillat ein nahezu azeotropes Gemisch. Die bei geringerem Betriebsdruck arbeitende zweite oder weitere(n) Destillationskolonne(n) wird in ganz besonders bevorzugten Ausführungsformen vorzugsweise mit der Kondensationswärme des oder der Kopfkondensator(en) der ersten Destillationskolonne betrieben.

Beim Zweidruckverfahren macht man sich die Druckabhängigkeit der azeotropen Zusammensetzung eines Zweistoffgemisches zunutze. Bei einem Gemisch aus Reaktionsalkohol (Alkylalkohol) und Dialkylcarbonat, wie beispielsweise Methanol und Dimethylcarbonat, verschiebt sich die azeotrope Zusammensetzung mit zunehmendem Druck zu höheren Reaktionsalkoholgehalten. Führt man ein Gemisch dieser beiden Komponenten einer Kolonne (Dialkylcarbonatkolonne) zu, wobei der Reaktionsalkoholgehalt unterhalb der für den Betriebsdruck dieser Kolonne entsprechende azeotropen Zusammensetzung liegt, so erhält man als Destillat ein Gemisch mit nahezu azeotroper Zusammensetzung und als Sumpfprodukt nahezu reines Dialkylcarbonat. Das so erhaltende azeotrope Gemisch wird einer weiteren Destillationskolonne (Alkylalkoholkolonne) zugeführt. Diese arbeitet bei einem im Vergleich zur Dialkylcarbonatkolonne geringeren Betriebsdruck. Dadurch verschiebt sich die Lage des Azeotrops hin zu geringeren Reaktionsalkoholgehalten. Hierdurch ist es möglich, das in der Dialkylcarbonatkolonne erhaltene azeotrope Gemisch in ein Destillat mit nahezu azeotroper Zusammensetzung und nahezu reinem Reaktionsalkohol zu trennen. Das Destillat der Alkylalkoholkolonne wird wieder der Dialkylcarbonatkolonne an geeigneter Stelle zugeführt.

Der Betriebsdruck der Alkylalkoholkolonne wird vorzugsweise so gewählt, dass man diese mit der Abwärme der Dialkylcarbonatkolonne betreiben kann. Der Betriebsdruck liegt dabei zwischen 0,1 und 1 bar vorzugsweise zwischen 0,3 und 1 bar. Der Betriebsdruck der Dialkylcarbonatkolonne liegt im Bereich von 1 bis 50 bar, vorzugsweise zwischen 2 und 20 bar.

Ein weiteres bevorzugtes Verfahren zur Trennung von Azeotropen aus Reaktionsalkohol und Dimethylcarbonat ist das Hybridverfahren. Beim Hybridverfahren erfolgt die Trennung eines Zweistoffgemisches mittels einer Kombination aus Destillation und Membranverfahren. Dabei macht man sich die Tatsache zunutze, dass man die Komponenten aufgrund ihrer polaren Eigenschaften und ihres unterschiedlichen Molekulargewichts mittels Membranen zumindest teilweise voneinander trennen kann. Im Falle eines Gemisches aus Reaktionsalkohol und Dialkylcarbonat, wie beispielsweise Methanol und Dimethylcarbonat, erhält man bei Verwendung geeigneter Membranen mittels Pervarporation oder Dampfpermeation ein an Reaktionsalkohol reiches Gemisch als Permeat und ein an Reaktionsalkohol verarmtes Gemisch als Retentat. Führt man ein Gemisch dieser beiden Komponenten einer Kolonne (Dialkylcarbonatkolonne) zu, wobei der Reaktionsalkoholgehalt unterhalb der für den Betriebsdruck dieser Kolonne entsprechenden azeotropen Zusammensetzung liegt, so erhält man als Destillat ein Gemisch mit im Vergleich zum Zulauf deutlich erhöhtem Reaktionsalkoholgehalt und als Sumpfprodukt nahezu reines Dimethylcarbonat.

Im Falle eines Hybridverfahrens aus Destillation und Dampfpermeation wird das Destillat der Kolonne dampfförmig entnommen. Das so erhaltende dampfförmige Gemisch wird gegebenenfalls nach Überhitzung einer Dampfpermeation zugeführt. Diese wird so betrieben, dass man auf der Retentatseite nahezu den Betriebsdruck der Kolonne und auf der Permeatseite einen geringeren Druck einstellt. Der Betriebsdruck der Kolonne liegt dabei im Bereich von 1 bis 50 bar, bevorzugt zwischen 1 und 20 und besonders bevorzugt zwischen 2 und 10 bar. Der Druck auf der Permeatseite liegt zwischen 0,05 und 2 bar. Dabei erhält man auf der Permeatseite eine an Reaktionsalkohol reiche Fraktion mit einem Reaktionsalkoholgehalt von mindestens 70 Gew.-%, bevorzugt mindestens 90 Gew.-%, bezogen auf das Gesamtgewicht der Fraktion. Das Retentat, welches einen im Vergleich zum Destillat der Kolonne einen reduzierten Reaktionsalkoholanteil enthält, wird gegebenenfalls kondensiert und wieder der Destillationskolonne zugeführt.

Im Falle eines Hybridverfahrens aus Destillation und Pervaporation wird das Destillat der Kolonne flüssig entnommen. Das so erhaltende Gemisch wird gegebenenfalls nach Erhitzung einer Pervaporation zugeführt. Diese wird so betrieben, dass man auf der Retentatseite einen im Vergleich zur Kolonne identischen oder erhöhten Betriebsdruck auf der Permeatseite einen geringeren Druck einstellt. Der Betriebsdruck der Kolonne liegt dabei im Bereich von 1 bis 50 bar, bevorzugt zwischen 1 und 20 und besonders bevorzugt zwischen 2 und 10 bar. Der Druck auf der Permeatseite liegt zwischen 0,05 und 2 bar. Dabei erhält man auf der Permeatseite eine an Reaktionsalkohol reiche dampfförmige Fraktion mit einem Reaktionsalkoholgehalt von mindestens 70 Gew.-%, bevorzugt mindestens 90 Gew.-%, bezogen auf das Gesamtgewicht der Fraktion. Das flüssige Retentat, welches einen im Vergleich zum Destillat der Kolonne reduzierten Reaktionsalkoholanteil erhält, wird wieder der Destillationskolonne zugeführt. Durch die Verdampfung des Permeats wird Wärme benötigt, die gegebenenfalls nicht in ausreichendem Maße im Zulaufstrom zur Pervaporation enthalten ist. Daher kann eine Membrantrennung mittels Pervarporation gegebenenfalls mit zusätzlichen Wärmetauschern beheizt werden, wobei diese integriert oder gegebenenfalls zwischen mehreren hintereinander geschalteten Pervaporationsschritten angebracht sind.

Die Trennung von Dimethylcarbonat und Reaktionsalkohol erfolgt im Falle eines Hybridverfahrens besonders bevorzugt mittels einer Kombination aus Destillation und Dampfpermeation.

Unabhängig vom gewählten Verfahren zur Trennung von Dimethylcarbonat und Reaktionsalkohol werden die Verfahrensbedingungen, wie Druck und Temperatur vorteilhafterweise so gewählt, das die die durch Kondensation in dem oder den Kondensator(en) der weiteren Reaktionskolonne(n) und/oder dem oder den gegebenenfalls vorhandenen Zwischenkondensator(en) der ersten Reaktionskolonne gewonnene Kondensationswärme effektiv genutzt werden kann.

Das erfindungsgemäße Verfahren wird bevorzugt kontinuierlich durchgeführt.

Auf der Zulaufseite befindet sich oberhalb der Reaktionszone mindestens eine weitere Sektion (K₁TLO). Diese enthält keinen Katalysator oder im Falle einer homogen katalysierten Reaktion gegebenenfalls nur sehr geringe Mengen (< 1 Gew.-%), so dass die Umesterung in dieser Sektion nicht oder nur in geringem Maße stattfindet. Aufgabe dieser Sektion ist die Abtrennung des Alkylarylcarbonats, höher siedender Verbindungen und bevorzugt auch der aromatischen Hydroxyverbindung vom Dimethylcarbonat und Reaktionsalkohol.

Die Anzahl der theoretischen Trennstufen in Sektion K₁TLO liegt unabhängig von der Art der gewählten Einbauten zwischen 1 und 100, bevorzugt zwischen 5 und 50 und besonders bevorzugt zwischen 10 und 30.

Als Einbauten können Packungen, Füllkörper oder Böden oder Kombinationen aus verschiedenen Einbauten (z.B. strukturierte Packungen und Böden) eingesetzt werden. Bevorzugt werden strukturierte Packungen oder Füllkörper eingesetzt.

In einer besonderen Ausführungsform enthält diese Sektion (K₁TLO) auch noch einen oder mehrere Zwischenkondensatoren (K₁ICTLO_{1-N}). Durch die Verwendung von Zwischenkondensatoren kann die Kondensationswärme auf einem höheren Temperaturniveau abgeführt und somit zur Beheizung anderer Verfahrenabschnitte oder zur Erzeugung eines Heizmediums, wie beispielsweise Heizdampf, verwendet werden.

Desweiteren befindet sich auf der Zulaufseite mindestens eine weitere Sektion unterhalb der Reaktionszone (K₁TLU). Aufgabe dieser Sektion ist die Abtrennung des Reaktionsalkohols und die Abreicherung des Dimethylcarbonats.

Die Anzahl der theoretischen Trennstufen in Sektion K₁TLU liegt unabhängig von der Art der gewählten Einbauten zwischen 1 und 100, bevorzugt zwischen 5 und 50 und besonders bevorzugt zwischen 10 und 30.

Als Einbauten können Packungen, Füllkörper oder Böden oder Kombinationen aus verschiedenen Einbauten (z.B. Packungen und Böden) eingesetzt werden. Bevorzugt werden Böden eingesetzt.

Bei Verwendung eines homogenen Umesterungskatalysators findet in dieser Sektion auch noch die Reaktion statt. In einer besonderen Ausführungsform des Verfahrens befinden sich in dieser Sektion auch noch Zwischenverdampfer (K₁ETLU_{1-N}).

Auf der Entnahmeseite befinden sich mindestens zwei Sektionen. Oberhalb der Entnahme befindet sich mindestens eine Sektion (K₁TRO). Diese dient zur Abreicherung an Reaktionsalkohol, welcher in der am oberen Ende dieser Sektionen aufgegebenen Flüssigkeit (11) enthalten ist.

Die Anzahl der theoretischen Trennstufen in Sektion K₁TRO liegt unabhängig von der Art der gewählten Einbauten zwischen 1 und 100, bevorzugt zwischen 5 und 50 und besonders bevorzugt zwischen 10 und 30.

Als Einbauten können Packungen, Füllkörper oder Böden oder Kombinationen aus verschiedenen Einbauten (z.B. Packungen und Böden) eingesetzt werden. Bevorzugt werden Böden eingesetzt.

In einer besonderen Ausführungsform enthält diese Sektion noch einen oder mehrere Zwischenverdampfer (K₁ETRO_{1-N}).

Unterhalb der Entnahme befindet sich mindestens eine weitere Sektion (K₁TRU). Diese dient zur Abreicherung von Komponenten, deren Siedepunkt oberhalb dem des Dimethylcarbonats liegt und welche im Dampfstrom (13) aus dem Abtriebsteil enthalten sind.

Die Anzahl der theoretischen Trennstufen in Sektion K₁TRO liegt unabhängig von der Art der gewählten Einbauten zwischen 1 und 100, bevorzugt zwischen 5 und 50 und besonders bevorzugt zwischen 10 und 30.

Als Einbauten können Packungen, Füllkörper oder Böden oder Kombinationen aus verschiedenen Einbauten (z.B. Packungen und Böden) eingesetzt werden. Bevorzugt werden Böden eingesetzt.

In einer besonderen Ausführungsform enthält diese Sektion noch einen oder mehrere Zwischenkondensatoren (K₁ICTRU_{1-N}).

Die Entnahme des Seitenstroms (5) erfolgt flüssig.

Im Verstärkungsteil (K₁VT) der Kolonne, welcher aus mindestens einer Sektion besteht und welcher in besonderen Ausführungsformen über einen oder mehrere Zwischenkondensatoren verfügt (K₁ICVT_{1-N}), erfolgt die Aufkonzentrierung des Reaktionsalkohols.

Die Anzahl der theoretischen Trennstufen im Verstärkungsteil liegt unabhängig von der Art der gewählten Einbauten zwischen 1 und 100, bevorzugt zwischen 5 und 50 und besonders bevorzugt zwischen 10 und 30.

Als Einbauten können Packungen, Füllkörper oder Böden oder Kombinationen aus verschiedenen Einbauten (z.B. Packungen und Böden) eingesetzt werden. Bevorzugt werden Füllkörper oder strukturierte Packungen eingesetzt.

Bei Verwendung von Diethylcarbonat (DEC) als einem nicht beanspruchten Dialkylcarbonat, kann der Reaktionsalkohol am Kopf der Kolonne in nahezu reiner Form entnommen werden. Im Falle des Dialkylcarbonats Dimethylcarbonat (DMC) erhält man am Kopf der Kolonne ein Methanol-Dimethylcarbonat-Gemisch mit nahezu azeotroper Zusammensetzung.

Der aus dem Verstärkungsteil (K₁VT) kommende Dampf (7) wird teilweise oder vollständig kondensiert. Das anfallende Kondensat wird teilweise oder vollständig als Rücklauf (8) auf die Kolonne aufgegeben. Das Destillat wird somit teilweise dampfförmig (3) und teilweise flüssig (4) entnommen. Die Art der Entnahme richtet sich nach der sich gegebenenfalls anschließenden weiteren Aufarbeitung des /der Destillatstromes /-ströme. Der Anteil des dampfförmigen Destillates (3) am Gesamtdestillatstrom, also die Gesamtmenge der Ströme 3 und 4, kann zwischen 0 und 100 % betragen. Das Rücklaufverhältnis errechnet sich aus dem Verhältnis aus Rücklaufmenge zu Gesamtdestillatstrom und liegt zwischen 0.5 und 50 bevorzugt zwischen 1 und 30 und besonders bevorzugt zwischen 5 und 20.

Die aus dem Verstärkungsteil ablaufende Flüssigkeit wird in zwei Ströme aufgeteilt. Ein Teilstrom (10) wird auf die obere Sektion auf der Zulaufseite (K₁TLO), die restliche Flüssigkeit (11) am oberen Ende der Entnahmeseite (K₁TRO) aufgegeben. Der Anteil der auf die Zulaufseite aufgegebenen Flüssigkeit bezogen auf die Gesamtmenge der aus dem Verstärkungsteil (K₁VT) ablaufenden Flüssigkeit beträgt 5 bis 95 %, bevorzugt 10 bis 80 % und besonders bevorzugt 25 bis 50%.

Im Abtriebsteil (K₁AT) der RWTK werden Dimethylcarbonat und Reaktionsalkohol abgereichert. In einer bevorzugten Ausführung des Verfahrens wird die Reaktive Trennwandkolonne so betrieben, dass im Abtriebsteil der Kolonne nur noch geringe Mengen an Reaktionsalkohol durch Umesterung entstehen. Im Abtriebsteil und im Sumpf der Kolonne entstehender Reaktionsalkohol gelangt teilweise in die Seitenstromentnahme. Der Betrieb der Reaktiven Trennwandkolonne soll bevorzugt so erfolgen, dass der Gehalt an Reaktionsalkohol im Seitenstrom kleiner 5 Gew.-%, bevorzugt kleiner 1 Gew.-% und besonders bevorzugt kleiner 0,5 Gew.-% ist.

Die Anzahl der theoretischen Trennstufen im Abtriebsteil liegt unabhängig von der Art der gewählten Einbauten zwischen 1 und 80, bevorzugt zwischen 5 und 40 und besonders bevorzugt zwischen 5 und 20.

Als Einbauten können Packungen, Füllkörper oder Böden oder Kombinationen aus verschiedenen Einbauten (z.B. Packungen und Böden) eingesetzt werden. Bevorzugt werden Böden eingesetzt.

In einer besonderen Ausführungsform sind im Abtriebsteil der RWTK einer oder mehrere Zwischenverdampfer vorgesehen (K₁EAT_{1-N}).

Der aus dem Abtriebsteil (K₁AT) austretende Dampf wird in einen Dampfstrom zur Zulaufseite (12) und einen Dampfstrom zur Entnahmeseite (13) aufgeteilt. Der Anteil des Dampfstromes zur Zulaufseite (12) am gesamten Dampfstrom, also der Summe aus Strom 12 und 13, beträgt 5 bis 95 %, bevorzugt 10 bis 80 % und besonders bevorzugt 30 bis 75 %.

Die aus dem Abtriebsteil (K₁AT) ablaufende Flüssigkeit (9) wird in einem oder mehreren Verdampfer weiter aufkonzentriert. In einer besonderen Ausführungsform dient der oder die Verdampfer K₁E_{1-N} als einzige Wärmezufuhr für die Reaktion K1.

Die Sumpftemperatur liegt dabei zwischen 100 und 300 °C, bevorzugt zwischen 150 und 280 und besonders bevorzugt zwischen 200 und 250 °C.

Das Sumpfprodukt der Reaktion K1 (6) enthält aromatische Hydroxyverbindung, Alkylaraylcarbonat, Diarylcarbonat, Dimethylcarbonat, Katalysator und Nebenverbindungen (Definition Nebenverbindungen: Verbindungen, die als Verunreinigungen in den Edukten enthalten sind oder als unerwünschte Nebenprodukte bei der Reaktion entstehen) und wird in einer bevorzugten Ausführungsform mindestens einer weiteren Reaktionsstufe zugeführt, in der das Alkylarylcarbonat bevorzugt durch Disproportionierung zum Diarylcarbonat weiterreagiert.

Bei Dimethylcarbonat als Dialkylcarbonat erhält man als Destillat (Strom 3 und/oder 4) ein Gemisch aus Methanol und Dimethylcarbonat mit nahezu azeotroper Zusammensetzung. Um eine höhere Konzentration des Reaktionsalkohols zu ermöglichen ist mindestens ein weiterer Aufarbeitungsschritt erforderlich. Eine Möglichkeit zur weiteren Aufkonzentrierung sind Membranverfahren. Bevorzugte Membranverfahren sind die Pervaporation und die Dampfpermeation.

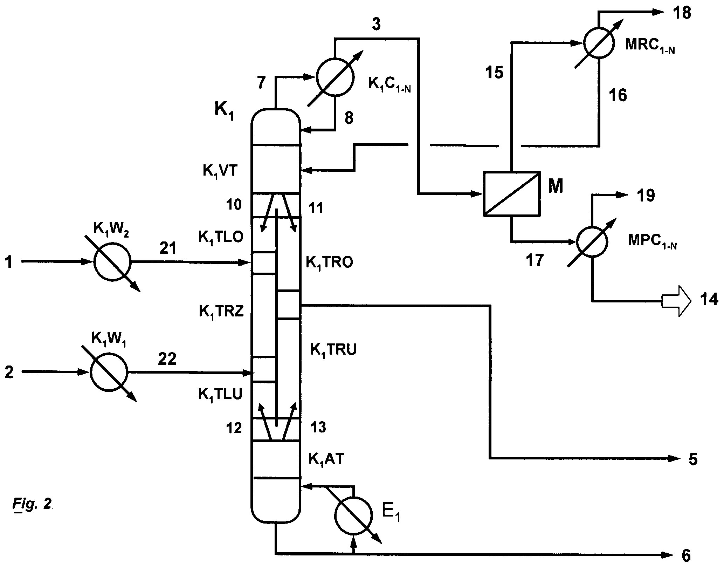

Eine solche Verfahrensvariante mit Dampfpermeation wird in Fig. 2 beispielhaft beschrieben.

Bei dieser besonderen Ausführungsform wird das Destillat (3) ausschließlich dampfförmig entnommen und einer Membrantrennung (M) mittels Pervaporation zugeführt. Als Permeat (17) erhält man eine Methanolreiche Fraktion mit mehr als 70 Gew.-% bevorzugt mehr als 90 Gew.-% Methanol. Als Retentat (15) erhält man eine Methanolarme Fraktion mit weniger als 60, bevorzugt weniger als 50 Gew.-% Methanol. Das Methanolreiche Permeat (17) wird kondensiert (Kondensator MPC_{1-N}) und aus dem Verfahren ausgeschleust. Das Retentat (15) wird in einer bevorzugten Ausführungsform kondensiert (Kondensator MRC_{1-N}) und wieder der Reaktiven Trennwandkolonne im Bereich des Verstärkungsteils (K₁VT) zugeführt.

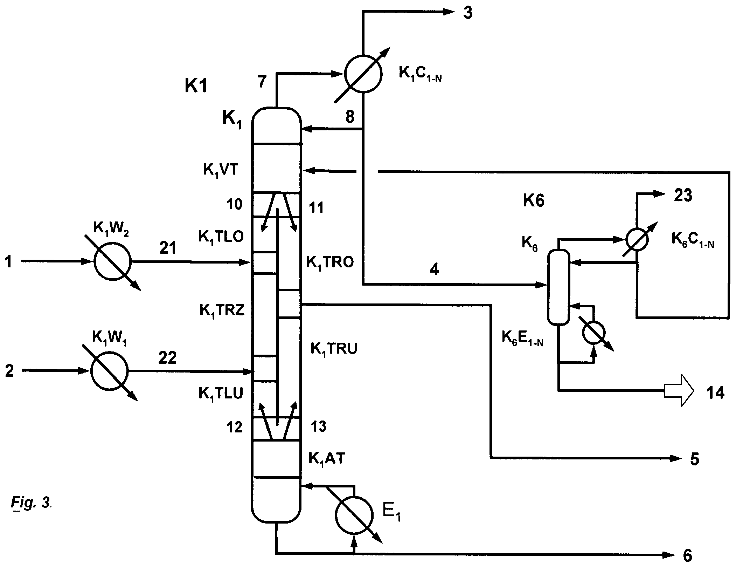

Eine weitere bevorzugte Ausführungsform zur weiteren Aufkonzentrierung des Methanols ist auf Fig. 3 abgebildet. Im diesen Falle wird das Destillat bevorzugt flüssig (4) entnommen. Im dampfförmigen Destillatstrom (3) sind Inerte (z.B. Stickstoff oder Kohlendioxid) und auch Nebenverbindungen mit Siedepunkten unterhalb dem des Methanols bzw. des Methanol/Dimethylcarbonat-Azeotrops enthalten.

Das flüssige Destillat (4) mit einer nahezu azeotropen Zusammensetzung wird einer weiteren Destillationskolonne (K₆) zugeführt, welche bei einem Druck unterhalb dem der Reaktiven Trennwandkolonne betrieben wird. Bevorzugt wird ein Druck kleiner 2 bar, bevorzugt kleiner 1 bar eingestellt. Aufgrund des geringeren Betriebsdruckes verschiebt sich die Lage des azeotropen Punktes hin zu geringeren Methanolgehalten. Man erhält eine Destillatfraktion mit nahezu azeotroper Zusammensetzung und eine Sumpffraktion mit reinem Methanol. Die Destillatfraktion wird wieder der Reaktiven Trennwandkolonne an geeigneter Stelle, bevorzugt im Verstärkungsteil (K₁VT) zugeführt.

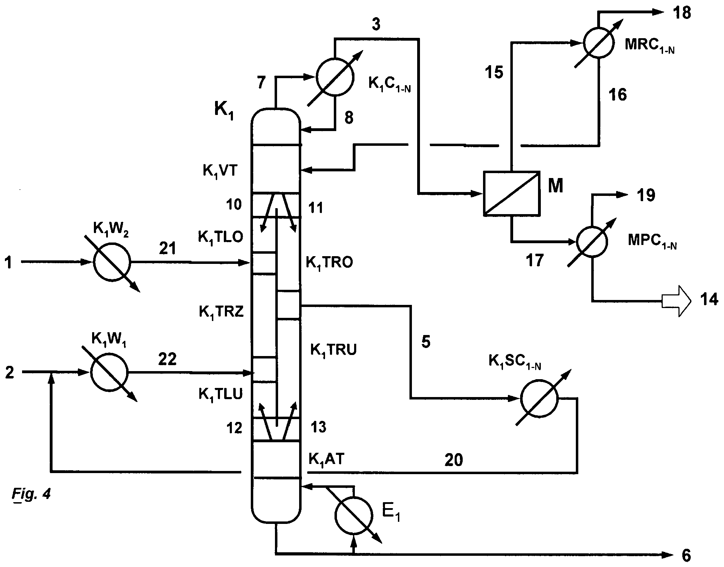

Fig. 4 beschreibt eine besondere, nicht beanspruchte Ausführungsform der reaktiven Trennwandkolonne (K₁) mit Auftrennung des gasförmigen Destillates in einer Membrantrennung. Im Unterschied zur Ausführung gemäß Abbildung 2 erfolgt die Entnahme des Dimethylcarbonathaltigen Seitenstromes (5) dampfförmig. Dieser wird kondensiert, gegebenenfalls mit einem weiteren Dimethylcarbonat-haltigen Strom (2) vermischt und anschließend, gegebenenfalls nach Verdampfung (Verdampfer K₁W₁) wieder der Reaktionszone auf der Zulaufseite der Reaktiven Trennwandkolonne zugeführt.

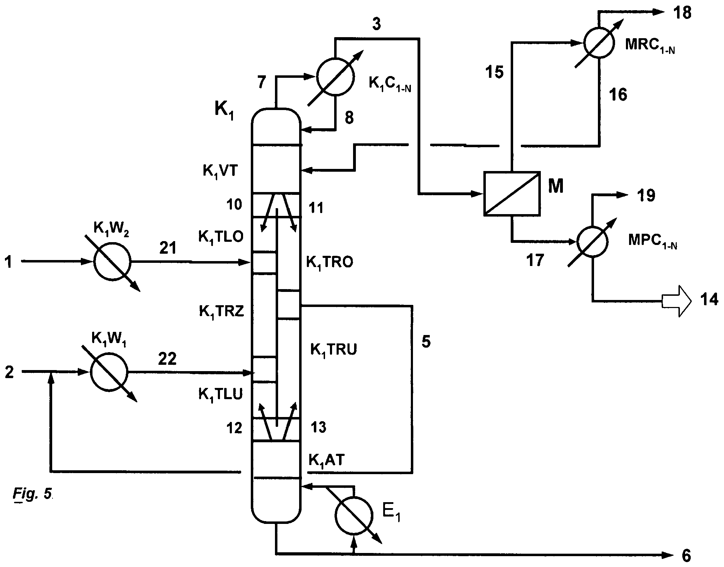

Fig. 5 beschreibt eine beanspruchte Ausführungsform der reaktiven Trennwandkolonne mit Auftrennung des gasförmigen Destillates in einer Membrantrennung wobei die Entnahme des Dimethylcarbonat-haltigen Seitenstroms (5) aus der reaktiven Trennwandkolonne als Flüssigkeit erfolgt. Der flüssige Seitenstrom wird gegebenenfalls mit einem weiteren Dimethylcarbonathaltigen Strom (2) vermischt und anschließend, gegebenenfalls nach Verdampfung wieder der Reaktionszone auf der Zulaufseite der Reaktiven Trennwandkolonne zugeführt.

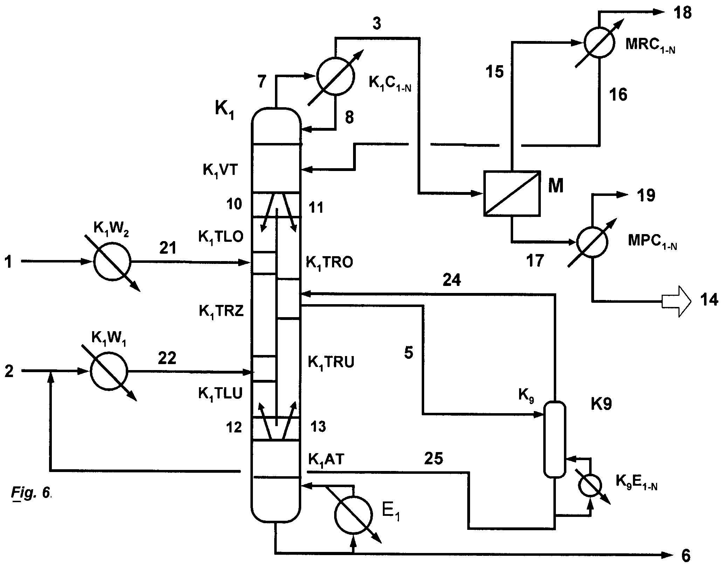

Fig. 6 beschreibt eine besondere Ausführungsform der reaktiven Trennwandkolonne mit Auftrennung des gasförmigen Destillates in einer Membrantrennung.

In diesem Falle wird der Seitenstrom flüssig entnommen und zwecks Abtrennung von eventuell noch enthaltenem Reaktionsalkohol einer weiteren Destillationskolonne zugeführt.

Bei entsprechender Reaktionsführung, insbesondere bei Verwendung eines homogenen Katalysators findet die Umesterung auch im Abtriebsteil (K₁AT) der Reaktiven Trennwandkolonne statt. Dadurch gelangt Reaktionsalkohol auch auf die Entnahmeseite der Reaktiven Trennwandkolonne und somit auch z.T. in flüssigen Seitenstrom. Bei hohen Anforderungen bezüglich des Restgehaltes an Reaktionsalkohol muss dieser in einem weiteren Schritt abgetrennt werden.

Dies kann wie in Abbildung 6 dargestellt dadurch erfolgen, dass man den flüssigen Seitenstrom einem Seitenstromstripper (K₉) zuführt. Dabei erhält man eine Dampffraktion (24) am Kopf der Strippkolonne, welcher wieder der Reaktiven Trennwandkolonne an geeigneter Stelle, bevorzugt oberhalb der flüssigen Seitenstromentnahme (5) zugeführt wird. Das Sumpfprodukt (25) der Strippkolonne (K₉) wird gegebenenfalls mit einem weiteren Dimethylcarbonat-haltigen Strom (2) vermischt und anschließend, gegebenenfalls nach Verdampfung wieder der Reaktionszone (K₁RZ) auf der Zulaufseite der Reaktiven Trennwandkolonne zugeführt.

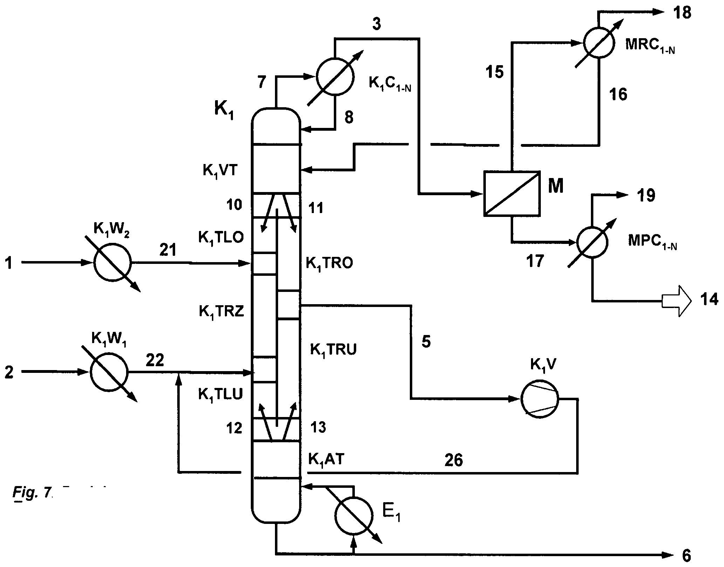

Fig. 7 beschreibt eine besondere Ausführungsform der reaktiven Trennwandkolonne mit Auftrennung des gasförmigen Destillates in einer Membrantrennung. In diesem Falle erfolgt eine dampfförmige Entnahme des Seitenstroms.

Anschließend wird dieser Seitenstrom mittels einer Vorrichtung zur Druckerhöhung (K₁V), bevorzugt mittels eines Verdichters oder Gebläses auf einen höheren Druck gebracht und gegebenenfalls nach Überhitzung und Vermischung mit einem weiteren dampfförmigen Dimethylcarbonat-haltigen Strom der Reaktionszone (K₁RZ) auf der Zulaufseite der Reaktiven Trennwandkolonne zugeführt.
Fig. 1 beschreibt einen ersten Umesterungsschritt mittels einer Reaktiven Trennwandkolonne ("im allgemeinen")
Fig. 2 beschreibt eine besondere Ausführungsform der reaktiven Trennwandkolonne mit Auftrennung des gasförmigen Destillates in einer Membrantrennung
Fig. 3 beschreibt eine besondere Ausführungsform der reaktiven Trennwandkolonne mit Auftrennung des flüssigen Destillates in einer Destillationskolonne
Fig. 4 beschreibt eine besondere Ausführungsform der reaktiven Trennwandkolonne mit Auftrennung des gasförmigen Destillates in einer Membrantrennung, Kondensation des dampfförmigen Seitenstroms und Vermischung des so erhaltenen Kondensat mit einem Eduktstrom.
Fig. 5 beschreibt eine beanspruchte Ausführungsform der reaktiven Trennwandkolonne mit Auftrennung des gasförmigen Destillates in einer Membrantrennung, Entnahme eines flüssigen Seitenstroms aus der reaktiven Trennwandkolonne und Vermischung dieses Seitenstroms mit einem Eduktstrom.
Fig. 6 beschreibt eine besondere Ausführungsform der reaktiven Trennwandkolonne mit Auftrennung des gasförmigen Destillates in einer Membrantrennung, Entnahme eines flüssigen Seitenstroms aus der reaktiven Trennwandkolonne, Abtrennung von eventuell noch enthaltenem Reaktionsalkohol in einer Destillationskolonne und Vermischung des Sumpfproduktes dieser Destillationskolonne mit einem Eduktstrom.
Fig. 7 beschreibt eine besondere Ausführungsform der reaktiven Trennwandkolonne mit Auftrennung des gasförmigen Destillates in einer Membrantrennung, Verdichtung des dampfförmigen Seitenstroms und Vermischung des so erhaltenen überhitzen Dampfes mit einem erhitzten und gegebenenfalls teilweise verdampften Eduktstroms.
Fig. 8 beschreibt die Verfahrensstufe mit Kondensation in einem integrierten Kondensator.
Fig. 9 beschreibt die Verfahrensstufe mit Kondensation in einem Packungsbett.

Die Figuren dienen der beispielhaften Erläuterung der Erfindung und sind nicht als Beschränkung aufzufassen.

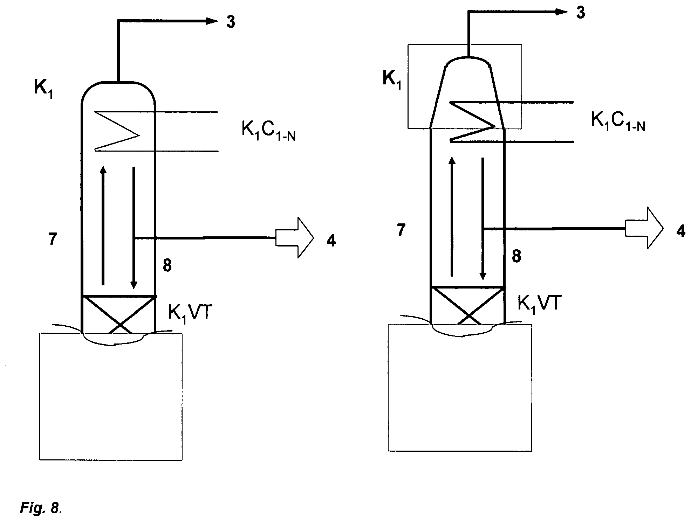

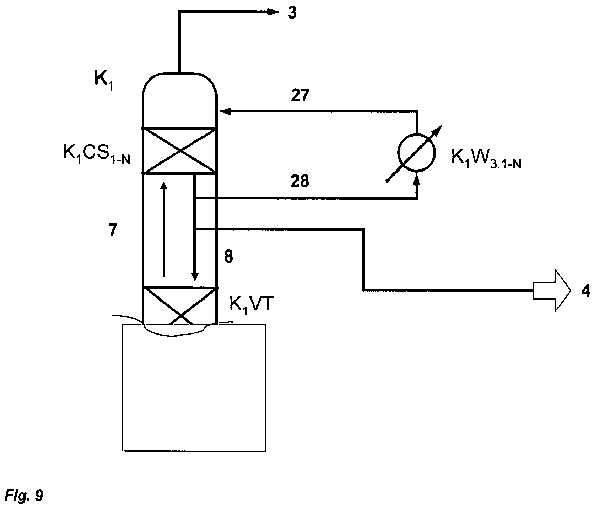

In den Fig. 1 bis 9 bedeuten
- K1: Verfahrensabschnitt Reaktion
- K₁:: Reaktive Trennwandkolonne
- K₁C_{1-N}: Kondensator(en) 1-N
- K₁E_{1-N}: Verdampfer 1 bis N
- K₁IC_{1-N}: Zwischenkondensator(en) 1 bis N
- T: Trennwand
- K₁VT_{1-N}: Verstärkungsteil (gegebenenfalls mehrere / 1-N Sektionen)
- K₁W_{1.1-N}: Vorwärmer/Verdampfer/Überhitzer für Dialkylcarbonat enthaltenden Strom
- K₁W_{2.1-N}: Vorwärmer/Verdampfer für Eduktstrom mit aromatischer Hydroxyverbindung
- K₁TRZ: Reaktionszone (Zulaufseite der Trennwand
- K₁E_TRZ_{1-N}: Zwischenverdampfer 1 bis N im Bereich der Reaktionszone
- K₁TLO: Sektion auf Zulaufseite oberhalb der Reaktionszone
- K₁ICTLO_{1-N}: Zwischenkondensator(en) 1-N in K₁TLO
- K₁TLU: Sektion auf Zulaufseite unterhalb der Reaktionszone
- K₁ETLU_{1-N}: Zwischenverdampfer 1-N in K₁TLU
- K₁TRO: Sektion auf Entnahmeseite oberhalb der Entnahme
- K₁ETLO_{1-N}: Zwischenverdampfer 1-N in K₁TRO
- K₁TRU: Sektion auf Entnahmeseite unterhalb der Entnahme
- K₁ICTRU_{1-N}: Zwischenkondensator(en) 1-N in K₁TRU
- K₁AT: Abtriebsteil
- K₁E_AT_{1-N}: Zwischenverdampfer 1-N im Abtriebsteil
- K₁SC_{1-N}: Seitenstromkondensator(en)
- K₁V: Seitenstromverdichter

- K6: Verfahrensabschnitt zur Destillation des Reaktionsalkohols (RAK)
- K₆: Reaktionsalkohol-Destillationskolonne (RAKD)
- K₆C_{1-N}: Kondensator(en) 1 bis N
- K₆E_{1-N}: Verdampfer 1 bis N
- K₆VT: Verstärkungsteil der RAKD
- K₆AT: Abtriebsteil der RAKD

Besondere Ausführung: Hybridverfahren bestehend aus K1 und Membrantrennung
- M: Membrantrennung (Dampfpermeation oder Pervaporation)
- MRC: Kondensator für Retentat nach der Membrantrennung
- MPC: Kondensator für Permeat nach der Membrantrennung

- K9:: Verfahrensabschnitt zur destillativen Abtrennung von Reaktionsalkohol aus Dialkylcarbonat
- K₉:: Destillationskolonne
- K₉C _{1-N}:: Kondensator (gegebenenfalls mehrstufig)
- K₉E_{1-N}:: Verdampfer für Sumpfprodukt (gegebenenfalls mehrstufig)
- K₉VT:: Verstärkungsteil
- K₉AT:: Abtriebsteil

### Besondere Ausführungsform Kondensation am Beispiel des Verfahrensabschnitts K1

- K₁CS_{1-N}:: Kolonnensegment mit direkter Kondensation
- K_{N}W_{3.1-N}:: Wärmetauscher zur Kühlung eines Umlaufstromes für die Kondensation in K_{N}CS_{1-N}

Weiterhin sind in den Fig. 1 bis 9 die folgenden Stoffströme benannt
- 1: Eduktstrom enthaltend aromatische Hydroxyverbindung
- 2: Eduktstrom entaltend Dialkylcarbonat
- 3: Dampfförmiges Destillat der Reaktion K1
- 4: Flüssiges Destillat der Reaktion K1
- 5: Dampfförmiger oder flüssiger Seitenstrom der K₁
- 6: Sumpfprodukt der Reaktion K1
- 7: Dampfstrom am Kopf der K₁ zum (zu den) Kondensator(en) K₁C_{1-N}
- 8: Rücklauf der K₁
- 9: Flüssiger Ablauf des Abtriebsteils der K₁
- 10: Flüssiger Ablauf des Verstärkungsteils der K₁ zur Zulaufseite der Trennwand
- 11: Flüssiger Ablauf des Verstärkungsteils der K₁ zur Entnahmeseite der Trennwand
- 12: Dampfstrom aus dem Abtriebsteil der K₁ zur Zulaufseite der Trennwand
- 13: Dampfstrom aus dem Abtriebsteil der K₁ zur Entnahmeseite der Trennwand
- 14: Aufkonzentrierter Reaktionsalkohol
- 15: Retentat der Membrantrennung (M) zum Kondensator (MRC_{1-N})
- 16: Flüssiges Retentat zur Reaktion (K1)
- 17: Permeat der Membrantrennung (M) zum Kondensator (MPC_{1-N})
- 18: Restlicher Dampfstrom nach Kondensator (MRC_{1-N})
- 19: Restlicher Dampfstrom nach Kondensator (MPC_{1-N})
- 20: Dialkylcarbonat enthaltender flüssiger Strom zur Reaktion K1
- 21: Aromatische Hydroxyverbindung enthaltender Strom zur K₁
- 22: Dialkylcarbonat enthaltender Strom zur K₁
- 23: Restlicher Dampfstrom nach K₆C_{1-N}
- 24: Dampfstrom am Kopf der K₉
- 25: Sumpfprodukt des Verfahrensabschnitt K9
- 26: Dialkylcarbonat enthaltender überhitzter Dampfstrom nach Verdichter K₁V zur K₁
- 27: Externer Kreislauf bei Kondensation in einem Kolonnensegment vor Kühlung
- 28: Externer Kreislauf bei Kondensation in einem Kolonnensegment nach Kühlung

### Beispiel

### Beispiel 1

In eine reaktive Trennwandkolonne (K₁) bestehend aus einem Verstärkungsteil (K₁VT) mit 19 theoretischen Stufen, einem Abtriebsteil (K₁AT) mit 9 theoretischen Stufen, einer Trennwand, die die Kolonne zwischen Verstärkungs- und Abtriebsteil in eine Zulauf- und Entnahmeseite aufteilt, einer oberen Sektion auf der Zulaufseite (K₁TLO) mit 10 theoretischen Stufen, einer Reaktionszone (K₁TRZ) auf der Zulaufseite der Trennwand mit 30 Reaktionsböden (Hold-up/Boden: 12 1), einer weiteren Sektion auf der Zulaufseite (K₁TLU) unterhalb der Reaktionszone mit 10 theoretischen Stufen, einer oberen Sektion auf der Entnahmeseite (K₁TRO) oberhalb der Seitenstromentnahme (5) mit 15 theoretischen Stufen, einer unteren Sektion auf der Entnahmeseite (K₁TRU) unterhalb der Seitenstromentnahme (5) mit 15 theoretischen Stufen, und einem Abtriebsteil K₁AT mit 6 Böden (Hold-up: 12 1) werden 396,9 kg/h eines Gemisches (21) mit 85.91 Gew.-% Phenol, 9.32 Gew.-% Dimethylcarbonat, 3.22 Gew.-% Diphenylcarbonat, 1.55 Gew.-% Titantetraphenolat, am oberen Ende der Reaktionszone (K₁TRZ) zudosiert. Am unteren Ende der Reaktionszone (K₁RZ) werden 539 kg/h eines Dampfgemisches (22) aus 98.9 Gew.-% Dimethylcarbonat, 0.9 Gew.-% Phenol, 0.2 Gew.-% Anisol zugeführt.

Die reaktive Trennwandkolonne wird bei einem Kopfdruck 3,6 bar (absolut) und einem Rücklaufverhältnis von 18 betrieben.

Dabei werden 32 % der aus dem Verstärkungsteil ablaufenden Flüssigkeit (10) der oberen Sektion der Zulaufseite (K₁TLO) zugeführt, was einer Menge von 317 kg/h entspricht. Die restliche Flüssigkeit (11) wird am oberen Ende der Entnahmeseite (K₁TRO) aufgegeben.

Im Sumpf der Kolonne wird eine Temperatur von 230 °C eingestellt, wobei als Sumpfverdampfer (K₁E₁) ein Kettle-Type-Verdampfer verwendet wird. Als Heizmedium wird Heizdampf (40 bara) verwendet.

Der aus dem Abtriebsteil austretende Dampf wird gleichmäßig auf Zulauf- und Entnahmeseite verteilt.

Das Destillat (3) wird ausschließlich dampfförmig entnommen. Man erhält 50 kg/h eines dampfförmigen Destillatstromes mit 68 Gew.-% Methanol und 31.9 Gew.-% Dimethylcarbonat. Die azeotrope Zusammensetzung beim angegebenen Betriebsdruck liegt bei 76 Gew.-%.

Im Seitenstrom (5) werden 515 kg/h mit 91,3 Gew.-% Dimethylcarbonat 8,5 Gew.-% Phenol und 0,2 Gew.-% Methanol als Flüssigkeit entnommen.

Ferner erhält man als Sumpfprodukt der Reaktion K1 394 kg/h eines Produktgemisches (6) mit 62.2 Gew.-% Phenol, 20.6 Gew.-% Methylphenylcarbonat, 5.3 Gew.-% Diphenylcarbonat, 10 Gew.-% Dimethylcarbonat, 0.3 Gew.-% Anisol und 1.6 Gew.-% Titantetraphenolat.

### Beispiel 2

Es wird die gleiche Reaktive Trennwandkolonne, wie bereits in Beispiel 1 beschrieben, verwendet. Am oberen Ende der Reaktionszone (K₁TRZ) werden 396,9 kg/h eines Gemisches (21) mit 85.91 Gew.-% Phenol, 9.32 Gew.-% Dimethylcarbonat, 3.22 Gew.-% Diphenylcarbonat, 1.55 Gew.-% Titantetraphenolat zudosiert.

Am unteren Ende der Reaktionszone (K₁RZ) werden 312,9 kg/h eines um 10 °C überhitzten Dampfgemisches (22) aus 86.1 Gew.-% Dimethylcarbonat, 13.7 Gew.-% Phenol und 0.2 Gew.-% Methanol zugeführt.

Die reaktive Trennwandkolonne wird bei einem Kopfdruck 3,6 bar (absolut) und einem Rücklaufverhältnis von 14.2 betrieben.

Dabei werden 37 % (202 kg/h) der aus dem Verstärkungsteil ablaufenden Flüssigkeit (10) der oberen Sektion der Zulaufseite (K₁TLO) zugeführt. Die restliche Flüssigkeit (11) wird am oberen Ende der Entnahmeseite (K₁TRO) aufgegeben.

Im Sumpf der Kolonne wird eine Temperatur von 230 °C eingestellt, wobei als Sumpfverdampfer (K₁E₁) ein Kettle-Type-Verdampfer verwendet wird. Als Heizmedium wird Heizdampf (40 bara) verwendet.

Der aus dem Abtriebsteil austretende Dampf wird gleichmäßig auf Zulauf- und Entnahmeseite verteilt.

Das Destillat (3) wird ausschließlich dampfförmig entnommen. Man erhält 20,5 kg/h eines dampfförmigen Destillatstromes mit 68 Gew.-% Methanol und 31.9 Gew.-% Dimethylcarbonat. Die azeotrope Zusammensetzung beim angegebenen Betriebsdruck liegt bei 76 Gew.-%.

Im Seitenstrom (5) werden 263 kg/h mit 83.4 Gew.-% Dimethylcarbonat, 16.3 Gew.-% Phenol und 0.3 Gew.-% Methanol als Flüssigkeit entnommen.

Der Seitenstrom wird mit einem weiteren Dimethylcarbonat-Strom (2) mit einer Mengen von 50 kg/h und einem Dimethylcarbonat-Gehalt von 100 % vermischt, verdampf, überhitzt und wieder der Reaktiven Trennwandkolonne unterhalb der Reaktionszone (K₁RZ) zugeführt.

Ferner erhält man als Sumpfprodukt der Reaktion K1 426.4 kg/h eines Produktgemisches (6) mit 70.1 Gew.-% Phenol, 15.3 Gew.-% Methylphenylcarbonat, 3.4 Gew.-% Diphenylcarbonat, 9.6 Gew.-% Dimethylcarbonat, 0.02 Gew.-% Anisol und 1.44 Gew.-% Titantetraphenolat.

### Beispiel 3

Es wird die gleiche Reaktive Trennwandkolonne, wie bereits in Beispiel 1 und 2 beschrieben, verwendet. Am oberen Ende der Reaktionszone (K₁TRZ) werden 396,9 kg/h eines Gemisches (21) mit 85.91 Gew.-% Phenol, 9.32 Gew.-% Dimethylcarbonat, 3.22 Gew.-% Diphenylcarbonat, 1.55 Gew.-% Titantetraphenolat zudosiert.

Am unteren Ende der Reaktionszone (K₁RZ) werden 386.2 kg/h eines um 10 °C überhitzten Dampfgemisches (22) aus 89.6 Gew.-% Dimethylcarbonat, 10.2 Gew.-% Phenol und 0.2 Gew.-% Methanol zugeführt.

Die reaktive Trennwandkolonne wird bei einem Kopfdruck 3,6 bar (absolut) und einem Rücklaufverhältnis von 10.4 betrieben.

Dabei werden 37 % (258.2 kg/h) der aus dem Verstärkungsteil ablaufenden Flüssigkeit (10) der oberen Sektion der Zulaufseite (K₁TLO) zugeführt. Die restliche Flüssigkeit (11) wird am oberen Ende der Entnahmeseite (K₁TRO) aufgegeben.

Im Sumpf der Kolonne wird eine Temperatur von 230 °C eingestellt, wobei als Sumpfverdampfer (K₁E₁) ein Kettle-Type-Verdampfer verwendet wird. Als Heizmedium wird Heizdampf (40 bara) verwendet.

Der aus dem Abtriebsteil austretende Dampf wird gleichmäßig auf Zulauf- und Entnahmeseite verteilt.

Das Destillat (3) wird ausschließlich dampfförmig entnommen. Man erhält 33 kg/h eines dampfförmigen Destillatstromes mit 68 Gew.-% Methanol und 31.7 Gew.-% Dimethylcarbonat. Die azeotrope Zusammensetzung beim angegebenen Betriebsdruck liegt bei 76 Gew.-%.

Im Seitenstrom (5) werden 336.2 kg/h mit 88.1 Gew.-% Dimethylcarbonat, 11.7 Gew.-% Phenol und 0.2 Gew.-% Methanol als Flüssigkeit entnommen.

Der Seitenstrom wird mit einem weiteren Dimethylcarbonat-Strom (2) mit einer Mengen von 50 kg/h und einem Dimethylcarbonat-Gehalt von 100 % vermischt, verdampf, überhitzt und wieder der Reaktiven Trennwandkolonne unterhalb der Reaktionszone (K₁RZ) zugeführt.

Als Sumpfprodukt der Reaktion K1 erhält man 336.2 kg/h eines Gemisches (6) mit 68.15 Gew.-% Phenol, 16.71 Gew.-% Methylphenylcarbonat, 3.9 Gew.-% Diphenylcarbonat, 9.8 Gew.-% Dimethylcarbonat, 0.01 Gew.-% Anisol und 1.43 Gew.-% Titantetraphenolat.

Dabei wird das dampfförmige Destillat einer Membrantrennung (M) mittels Pervaporation zugeführt. Als Retentat erhält man 16.2 kg/h eines dampfförmigen Stromes mit 40 Gew.-% Methanol, welches nach erfolgter Kondensation im oberen Drittel des Verstärkungsteils (K₁VT) der Reaktiven Trennwandkolonne zugeführt wird.

Als Permeat erhält man eine an Methanol reiche Fraktion (17) mit 95 Gew.-% Methanol.

### Beispiel 4

Es wird die gleiche Kombination aus Reaktiver Trennwandkolonne und Pervaporation, wie bereits in Beispiel 3 beschrieben, verwendet.

Zusätzlich hierzu wird der flüssige Seitenstrom (5) der reaktiven Trennwandkolonne (K₁) einem Seitenstromstripper (K₉) mit lediglich einem Abtriebsteil, enthaltend 14 theoretische Stufen zugeführt. Die Seitenstromstrippung hat keinen Kondensator und arbeitet bei einem Betriebsdruck von 4 bar, was etwa 10 mbar oberhalb des Druckes in der RTWK im Bereich der Seitenstromentnahme liegt. Die Beheizung der Seitenstromstrippung (K9) erfolgt mittels eines Umlaufverdampfers, der mit Heizdampf (6 bar) betrieben wird.

Am oberen Ende der Reaktionszone (K₁TRZ) werden 396,9 kg/h eines Gemisches (21) mit 85.91 Gew.-% Phenol, 9.32 Gew.-% Dimethylcarbonat, 3.22 Gew.-% Diphenylcarbonat, 1.55 Gew.-% Titantetraphenolat zudosiert.

Am unteren Ende der Reaktionszone (K₁RZ) werden 381.9 kg/h eines um 10 °C überhitzten Dampfgemisches (22) aus 89.7 Gew.-% Dimethylcarbonat, 10.1 Gew.-% Phenol und 0.2 Gew.-% Methanol zugeführt.

Die reaktive Trennwandkolonne wird bei einem Kopfdruck 3,6 bar (absolut) und einem Rücklaufverhältnis von 10.4 betrieben.

Dabei werden 37 % (258.8 kg/h) der aus dem Verstärkungsteil ablaufenden Flüssigkeit (10) der oberen Sektion der Zulaufseite (K₁TLO) zugeführt. Die restliche Flüssigkeit (11) wird am oberen Ende der Entnahmeseite (K₁TRO) aufgegeben.

Im Sumpf der Kolonne wird eine Temperatur von 230 °C eingestellt, wobei als Sumpfverdampfer (K₁E₁) ein Kettle-Type-Verdampfer verwendet wird. Als Heizmedium wird Heizdampf (40 bara) verwendet.

Der aus dem Abtriebsteil austretende Dampf wird gleichmäßig auf Zulauf- und Entnahmeseite verteilt.

Das Destillat (3) wird ausschließlich dampfförmig entnommen. Man erhält 33 kg/h eines dampfförmigen Destillatstromes mit 68 Gew.-% Methanol und 31.7 Gew.-% Dimethylcarbonat. Die azeotrope Zusammensetzung beim angegebenen Betriebsdruck liegt bei 76 Gew.-%.

Im Seitenstrom (5) werden 336.9 kg/h mit 88.3 Gew.-% Dimethylcarbonat, 11.44 Gew.-% Phenol und 0.23 Gew.-% Methanol als Flüssigkeit entnommen.

Das Sumpfprodukt der Seitenstrippung (K9) hat einen Methanolgehalt von weniger 0,2 Gew.-% und wird mit einem weiteren Dimethylcarbonat-Strom (2) mit einer Mengen von 50 kg/h und einem Dimethylcarbonat-Gehalt von 100 % vermischt, verdampf, überhitzt und wieder der Reaktiven Trennwandkolonne unterhalb der Reaktionszone (K₁RZ) zugeführt.

Als Kopfprodukt werden 5 kg/h dampfförmig entnommen und wieder der Reaktiven Trennwandkolonne oberhalb der Seitenstromentnahme (5) zugeführt.

Als Sumpfprodukt der Reaktion K1 erhält man 430.1 kg/h eines Gemisches (6) mit 68.15 Gew.-% Phenol, 16.71 Gew.-% Methylphenylcarbonat, 3.9 Gew.-% Diphenylcarbonat, 9.8 Gew.-% Dimethylcarbonat, 0.01 Gew.-% Anisol und 1.43 Gew.-% Titantetraphenolat.

Dabei wird das dampfförmige Destillat einer Membrantrennung (M) mittels Pervaporation zugeführt. Als Retentat erhält man 16.2 kg/h eines dampfförmigen Stromes mit 40 Gew.-% Methanol, welches nach erfolgter Kondensation im oberen Drittel des Verstärkungsteils (K₁VT) der Reaktiven Trennwandkolonne zugeführt wird.

Als Permeat erhält man eine an Methanol reiche Fraktion (17) mit 95 Gew.-% Methanol.

### Beispiel 5

Es wird die gleiche Kombination aus Reaktiver Trennwandkolonne, Pervaporation, und Seitenstrippung wie bereits in Beispiel 4 beschrieben, verwendet.

Allerdings wird die Reaktive Trennwandkolonne bei einem erhöhten Rücklaufverhältnis von 13,4 betrieben

Am oberen Ende der Reaktionszone (K₁TRZ) werden 396,9 kg/h eines Gemisches (21) mit 85.91 Gew.-% Phenol, 9.32 Gew.-% Dimethylcarbonat, 3.22 Gew.-% Diphenylcarbonat, 1.55 Gew.-% Titantetraphenolat zudosiert.

Am unteren Ende der Reaktionszone (K₁RZ) werden 514,8 kg/h eines um 10 °C überhitzten Dampfgemisches (22) aus 94,4 Gew.-% Dimethylcarbonat, 5,4 Gew.-% Phenol und 0.2 Gew.-% Methanol zugeführt.

Dabei werden 38 % (377,7 kg/h) der aus dem Verstärkungsteil ablaufenden Flüssigkeit (10) der oberen Sektion der Zulaufseite (K₁TLO) zugeführt. Die restliche Flüssigkeit (11) wird am oberen Ende der Entnahmeseite (K₁TRO) aufgegeben.

Im Sumpf der Kolonne wird eine Temperatur von 230 °C eingestellt, wobei als Sumpfverdampfer (K₁E₁) ein Kettle-Type-Verdampfer verwendet wird. Als Heizmedium wird Heizdampf (40 bara) verwendet.

Der aus dem Abtriebsteil austretende Dampf wird gleichmäßig auf Zulauf- und Entnahmeseite verteilt.

Das Destillat (3) wird ausschließlich dampfförmig entnommen. Man erhält 32,7 kg/h eines dampfförmigen Destillatstromes mit 74 Gew.-% Methanol und 25,8 Gew.-% Dimethylcarbonat. Die azeotrope Zusammensetzung beim angegebenen Betriebsdruck liegt bei 76 Gew.-%.

Im Seitenstrom (5) werden 470,8 kg/h mit 93,9 Gew.-% Dimethylcarbonat, 5,9 Gew.-% Phenol und 0.2 Gew.-% Methanol als Flüssigkeit entnommen.

Das Sumpfprodukt der Seitenstrippung (K9) hat einen Methanolgehalt von weniger 0,2 Gew.-% und wird mit einem weiteren Dimethylcarbonat-Strom (2) mit einer Mengen von 59 kg/h, einem Dimethylcarbonat-Gehalt von 99,9 % und einem Methanolgehalt von 0,1 Gew.-% vermischt, verdampf, überhitzt und wieder der Reaktiven Trennwandkolonne unterhalb der Reaktionszone (K₁RZ) zugeführt.

Als Kopfprodukt der Seitenstrippung (K9) werden 15 kg/h dampfförmig entnommen und wieder der Reaktiven Trennwandkolonne oberhalb der Seitenstromentnahme (5) zugeführt.

Als Sumpfprodukt der Reaktion K1 erhält man 435,7 kg/h eines Gemisches (6) mit 65.2 Gew.-% Phenol, 18.84 Gew.-% Methylphenylcarbonat, 4.6 Gew.-% Diphenylcarbonat, 9.94 Gew.-% Dimethylcarbonat, 0.01 Gew.-% Anisol und 1.41 Gew.-% Titantetraphenolat.

Dabei wird das dampfförmige Destillat einer Membrantrennung (M) mittels Pervaporation zugeführt. Als Retentat erhält man 12,5 kg/h eines dampfförmigen Stromes mit 40 Gew.-% Methanol, welches nach erfolgter Kondensation im oberen Drittel des Verstärkungsteils (K₁VT) der Reaktiven Trennwandkolonne zugeführt wird.

Als Permeat erhält man eine an Methanol reiche Fraktion (17) mit 95 Gew.-% Methanol.

## Patentansprüche

1. Verfahren zur Herstellung von Alkylarylcarbonaten und/oder Diarylcarbonaten aus Dimethylcarbonat und aromatischen Hydroxyverbindungen in Gegenwart eines Katalysators mittels einer Reaktivdestillationskolonne, die oberhalb des Abtriebsteils (K₁AT) und unterhalb eines Verstärkungsteils (K₁VT) durch eine in Längsrichtung verlaufende Trennwand (T) die eine Quervermischung von Flüssigkeits- und/oder Brüdenströmen ganz oder teilweise verhindern, in eine Zulaufseite (Z), in der sich eine Reaktionszone befindet, und eine Entnahmeseite (E) unterteilt ist, **dadurch gekennzeichnet, dass**
der Zulaufseite ein die aromatische Hydroxyverbindung enthaltender Strom (21) und ein das Dimethylcarbonat enthaltender Strom (22) zugeführt werden und
aus der Entnahmeseite ein Dimethylcarbonat-haltiger Seitenstrom (5) flüssig entnommen, ggf. mit einem weiteren Dimethylcarbonat-haltigen Strom (2) vermischt, und anschließend der Reaktionszone auf der Zulaufseite der reaktiven Trennwandkolonne zugeführt wird,
und am Kopf das Dampfgemisch enthaltend Dimethylcarbonat und während der Reaktion gebildetem Alkylalkohol entnommen und in einer Membrantrennung aufgetrennt wird.

2. Verfahren gemäß Anspruch 1, **dadurch gekennzeichnet, dass** die Trennwand eine Quervermischung von Flüssigkeits- und/oder Brüdenströmen ganz verhindert.

3. Verfahren nach Anspruch 1 oder 2, **dadurch gekennzeichnet, dass** der die aromatische Hydroxyverbindung enthaltende Strom (21) oberhalb dieser Reaktionszone und der das Dimethylcarbonat enthaltende Strom (22) unterhalb der Reaktionszone zugeführt wird.

4. Verfahren nach einem der vorangehenden Ansprüche, **dadurch gekennzeichnet, dass** es sich bei der aromatischen Hydroxyverbindung um Phenolund bei dem Diarylcarbonat um Diphenylcarbonat handelt.

5. Verfahren nach einem der vorangehenden Ansprüche, **dadurch gekennzeichnet, dass** die Reaktion homogen katalysiert wird.

6. Verfahren nach einem der vorangehenden Ansprüche, **dadurch gekennzeichnet, dass** der die aromatische Hydroxyverbindung enthaltender Strom (21) den Umesterungskatalysator enthält.

7. Verfahren nach einem der vorangehenden Ansprüche, **dadurch gekennzeichnet, dass** der die aromatische Hydroxyverbindung enthaltender Strom (21) und / oder der das Dimethylcarbonat enthaltende Strom (22) dampfförmig oder erhitzt zugeführt werden.

8. Verfahren nach einem der vorangehenden Ansprüche, **dadurch gekennzeichnet, dass** der die aromatische Hydroxyverbindung enthaltende Strom flüssig oder nur mit geringem Dampfanteil eindosiert und der Dimethylcarbonat-haltige Strom dampfförmig oder überhitzt zugeführt wird.

9. Verfahren nach einem der vorangehenden Ansprüche, **dadurch gekennzeichnet, dass** sich auf der Zulaufseite oberhalb der Reaktionszone mindestens eine weitere Sektion (K1TLO) befindet, die keinen oder maximal 1 Gew.-% Katalysator enthält.

10. Verfahren nach Anspruch 9, **dadurch gekennzeichnet, dass** die Sektion (K1TLO) mit wenigstens einem Zwischenkondensator ausgestattet ist und die durch Kondensation in diesem Kondensator gewonnene Kondensationswärme direkt oder indirekt wieder in das Verfahren zurückgeführt wird.

11. Verfahren nach einem der vorangehenden Ansprüche, **dadurch gekennzeichnet, dass** sich auf der Zulaufseite unterhalb der Reaktionszone mindestens eine weitere Sektion (K1TLU) befindet.

12. Verfahren nach einem der Ansprüche 10 oder 11, **dadurch gekennzeichnet, dass** der oder die Zwischenkondensator(en) in die Kolonne integriert oder als separate(r) Zwischenkondensator(en) außerhalb der Kolonne ausgeführt ist (sind).

13. Verfahren nach einem der vorangehenden Ansprüche, **dadurch gekennzeichnet, dass** die Reaktion bei einer Temperatur von 100 bis 300°C und einem Druck von 0,5 bis 20 bar erfolgt.

14. Verfahren nach einem der vorangehenden Ansprüche, **dadurch gekennzeichnet, dass** das aus dem Verstärkungsteil (K1VT) kommende Destillat teilweise oder vollständig als Rücklauf auf die Kolonne aufgegeben, wobei das Rücklaufverhältnis bei von 0,5 bis 50 liegt.

## Claims

1. Process for preparing alkyl aryl carbonates and/or diaryl carbonates from dimethyl carbonates and aromatic hydroxy compounds in the presence of a catalyst by means of a reactive distillation column which is divided above the stripping section (K₁AT) and below an enrichment section (K₁VT) by a dividing wall (T) running in the longitudinal direction which entirely or partially prevent transverse mixing of liquid and/or vapour streams into a feed side (Z) in which a reaction zone is located and an offtake side (E), **characterized in that** a stream (21) containing the aromatic hydroxy compound and a stream (22) containing the dimethyl carbonate are fed to the feed side and
a side stream (5) containing dimethyl carbonate is taken off in liquid form from the offtake side, optionally mixed with a further stream (2) containing dimethyl carbonate and subsequently fed to the reaction zone on the feed side of the reactive dividing wall column,
and the vapour mixture containing dimethyl carbonate and alkyl alcohol formed during the reaction is taken off at the top and separated in a membrane separation.

2. Process according to Claim 1, **characterized in that** the dividing wall completely prevents transverse mixing of liquid and/or vapour streams.

3. Process according to Claim 1 or 2, **characterized in that** the stream (21) containing the aromatic hydroxy compound is fed in above this reaction zone and a stream (22) containing the dimethyl carbonate is fed in below the reaction zone.

4. Process according to any of the preceding claims, **characterized in that** the aromatic hydroxy compound is phenol and the diaryl carbonate is diphenyl carbonate.

5. Process according to any of the preceding claims, **characterized in that** the reaction is homogeneously catalyzed.

6. Process according to any of the preceding claims, **characterized in that** the stream (21) containing the aromatic hydroxy compound contains the transesterification catalyst.

7. Process according to any of the preceding claims, **characterized in that** the stream (21) containing the aromatic hydroxy compound and/or the stream (22) containing the dimethyl carbonate are fed in in gaseous or heated form.

8. Process according to any of the preceding claims, **characterized in that** the stream containing the aromatic hydroxy compound is introduced in liquid form or with only a small proportion of vapour and the stream containing dimethyl carbonate is fed in in gaseous or superheated form.

9. Process according to any of the preceding claims, **characterized in that** at lease one further section (K1TLO) which contains no catalyst or a maximum of 1% by weight of catalyst is present on the feed side above the reaction zone.

10. Process according to Claim 9, **characterized in that** the section K1TLO is equipped with at least one intermediate condenser and the heat of condensation obtained by condensation in this condenser is returned either directly or indirectly to the process.

11. Process according to any of the preceding claims, **characterized in that** at least one further section (K1TLU) is present on the feed side below the reaction zone.

12. Process according to either Claim 10 or 11, **characterized in that** the intermediate condenser(s) is (are) integrated into the column or present as separate intermediate condenser(s) outside the column.

13. Process according to any of the preceding claims, **characterized in that** the reaction is carried out at a temperature of from 100 to 300°C and a pressure of from 0.5 to 20 bar.

14. Process according to any of the preceding claims, **characterized in that** the distillate coming from the enrichment section (K1VT) is partly or completely introduced as runback into the column, with the reflux ratio being from 0.5 to 50.

## Revendications

1. Procédé de fabrication de carbonates d'alkylaryle et/ou de carbonates de diaryle à partir de carbonate de diméthyle et de composés hydroxy aromatiques en présence d'un catalyseur au moyen d'une colonne de distillation réactive, qui est divisée au-dessus de la zone de rectification (K₁AT) et en dessous d'une zone d'enrichissement (K₁VT) par une paroi de séparation (T) orientée dans la direction longitudinale, qui empêche en totalité ou en partie un mélange de courants de liquide et/ou de vapeur, en un côté de l'alimentation (2), dans lequel se trouve une zone de réaction, et un côté du soutirage (E), **caractérisé en ce que** un courant contenant le composé hydroxy aromatique (21) et un courant contenant le carbonate de diméthyle (22) sont introduits du côté de l'alimentation, et
un courant latéral contenant du carbonate de diméthyle (5) est soutiré sous forme liquide du côté du soutirage, éventuellement mélangé avec un autre courant contenant du carbonate de diméthyle (2), puis introduit dans la zone de réaction du côté de l'alimentation de la colonne à paroi de séparation réactive,
et le mélange de vapeur contenant du carbonate de diméthyle et l'alcool alkylique formé pendant la réaction est soutiré à la tête et séparé dans une séparation à membrane.

2. Procédé selon la revendication 1, **caractérisé en ce que** la paroi de séparation empêche en totalité un mélange de courants de liquide et/ou de vapeur.

3. Procédé selon la revendication 1 ou 2, **caractérisé en ce que** le courant contenant le composé hydroxy aromatique (21) est introduit au-dessus de cette zone de réaction et le courant contenant le carbonate de diméthyle (22) en dessous de la zone de réaction.

4. Procédé selon l'une quelconque des revendications précédentes, **caractérisé en ce que** le composé hydroxy aromatique est le phénol et le carbonate de diaryle est le carbonate de diphényle.

5. Procédé selon l'une quelconque des revendications précédentes, **caractérisé en ce que** la réaction est réalisée sous catalyse homogène.

6. Procédé selon l'une quelconque des revendications précédentes, **caractérisé en ce que** le courant contenant le composé hydroxy aromatique (21) contient le catalyseur de transestérification.

7. Procédé selon l'une quelconque des revendications précédentes, **caractérisé en ce que** le courant contenant le composé hydroxy aromatique (21) et/ou le courant contenant le carbonate de diméthyle (22) sont introduits sous forme vapeur ou chauffés.

8. Procédé selon l'une quelconque des revendications précédentes, **caractérisé en ce que** le courant contenant le composé hydroxy aromatique est introduit sous forme liquide ou contenant seulement une faible proportion de vapeur, et le courant contenant le carbonate de diméthyle est introduit sous forme vapeur ou surchauffé.

9. Procédé selon l'une quelconque des revendications précédentes, **caractérisé en ce qu'**au moins une section supplémentaire (K₁TLO), qui ne contient pas ou au plus 1 % en poids de catalyseur, se trouve du côté de l'alimentation au-dessus de la zone de réaction.

10. Procédé selon la revendication 9, **caractérisé en ce que** la section (K₁TLO) est équipée d'au moins un condensateur intermédiaire et la chaleur de condensation obtenue par condensation dans ce condensateur est recyclée directement ou indirectement dans le procédé.

11. Procédé selon l'une quelconque des revendications précédentes, **caractérisé en ce qu'**au moins une section supplémentaire (K₁TLU) se trouve du côté de l'alimentation sous la zone de réaction.

12. Procédé selon l'une quelconque des revendications 10 ou 11, **caractérisé en ce que** le ou les condensateurs intermédiaires sont configurés sous forme intégrée dans la colonne ou sous la forme de condensateurs intermédiaires séparés à l'extérieur de la colonne.

13. Procédé selon l'une quelconque des revendications précédentes, **caractérisé en ce que** la réaction a lieu à une température de 100 à 300 °C et à une pression de 0,5 à 20 bar.

14. Procédé selon l'une quelconque des revendications précédentes, **caractérisé en ce que** le distillat sortant de la zone d'enrichissement (K₁VT) est introduit en partie ou en totalité dans la colonne en tant que reflux, le taux de reflux étant de 0,5 à 50.
